(19)

Europäisches
Patentamt
European
Patent Office
Office européen
des brevets

(11) **EP 3 677 911 A2**

(12) **EUROPEAN PATENT APPLICATION**

(43) Date of publication:
08.07.2020 Bulletin 2020/28

(51) Int Cl.:
*G01N 33/574* (2006.01)    *A61K 39/00* (2006.01)

(21) Application number: 19183071.0

(22) Date of filing: 27.06.2019

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **03.01.2019 EP 19150179**

(71) Applicant: **Universität Basel**
**4001 Basel (CH)**

(72) Inventor: **The designation of the inventor has not
yet been filed**

(74) Representative: **Schulz Junghans**
**Patentanwälte PartGmbB**
**Großbeerenstraße 71**
**10963 Berlin (DE)**

(54) **USE OF NON-AGONIST LIGANDS FOR SUPPRESSION OF METASTASIS**

(57)    The present invention relates to a non-agonist ligand specifically binding to VCAM1 (CD106), CD49d, CD29, the IL6 receptor (CD126), IL6, the IL1β receptor (IL1R1), IL1β, F11R, ICAM1, or ITGB2 for use in treatment or prevention of metastatic cancer. The method further relates to a method for assigning a likelihood of developing metastasis to a patient.

EP 3 677 911 A2

**Description**

The present invention relates to a non-agonist ligand specifically binding to VCAM1 (CD106), CD49d, CD29, the IL6 receptor (CD126), IL6, the IL1β receptor (IL1R1), IL1β, F11R, ICAM1, or ITGB2 for use in treatment or prevention of metastatic cancer.

Background of the invention

A better understanding of the features that define the interplay between cancer cells and immune cells is key to identifying new cancer therapies. Yet, focus is often given to those interactions that occur within the primary tumor and its microenvironment, while the role of immune cells during cancer dissemination in patients remains largely uncharacterized. Circulating tumor cells (CTCs) are precursors of metastasis in several cancer types, and are occasionally found within the bloodstream in association with non-malignant cells such as white blood cells (WBCs). The identity and function of these CTC-associated WBCs, as well as the molecular features that define the interaction between WBCs and CTCs are unknown.

A relationship between this interaction and clinical outcomes has not been identified to date. If such relationship existed, and modulation of the interaction were proven to provide an actionable target to suppress metastasis, a novel approach to cancer treatment would be provided.

The objective of the present invention is to provide means and methods to treat or prevent metastatic cancer. This objective is attained by the subject-matter of the independent claims of the present specification.

Description

*Summary of the invention*

A first aspect of the invention relates to a non-agonist ligand specifically binding to a protein target selected from:

- VCAM1 (CD106) (Entrez Gene-ID: 7412, Uniprot: P19320),

- CD49d (Entrez Gene-ID: 3676, Uniprot: P13612),

- CD29 (Entrez Gene-ID: 3688, Uniprot: P05556),

- IL6 receptor (CD126) (Entrez Gene-ID: 3570, Uniprot: P08887),

- IL6 (Entrez Gene-ID: 3569, Uniprot: P05231),

- IL1β receptor (IL1R1) (Entrez Gene-ID: 3554, Uniprot: P14778),

- IL1β (Entrez Gene-ID: 3553, Uniprot: P01584),

- F11R (Entrez Gene-ID: 50848, Uniprot: Q9Y624),

- ICAM1 (Entrez Gene-ID: 3383, Uniprot: P05362), or

- ITGB2 (Entrez Gene-ID: 3689, Uniprot: P05107),

for use in treatment or prevention of metastatic cancer.

A second aspect of the invention relates to nucleic acid mediated direct or indirect downregulation or inhibition of the expression of any the above protein targets in treatment of metastasis.

A third aspect of the invention relates to a method of diagnosis, wherein a likelihood of developing metastasis is assigned to a patient if

- the patient has at least one detectable CTC-neutrophil cluster in the bloodstream; and/or
- a tumour of the patient is characterized by a mutation in gene *TLE1* selected from 1787G>A or 1509G>C; and/or
- a tumour of the patient is characterized by a mutation in genes belonging to the frequently mutated gene list of Table 3 in patients with CTC-neutrophil clusters and /or
- more than 40% of detected mutations in a tumour of the patient are C>T substitutions.

A fourth aspect of the invention relates to the use of the ligand or the nucleic acid molecule according to the invention in treatment of metastasis in a patient who has been assigned a high likelihood of developing metastasis by the diagnostic method provided herein.

*Terms and definitions*

Unless defined otherwise, all technical and scientific terms used herein have the same meaning as commonly understood by one of ordinary skill in the art (e.g., in cell culture, molecular genetics, nucleic acid chemistry, hybridization techniques and biochemistry). Standard techniques are used for molecular, genetic and biochemical methods (see generally, Sambrook et al., Molecular Cloning: A Laboratory Manual, 2d ed. (1989) Cold Spring Harbor Laboratory Press, Cold Spring Harbor, N.Y . and Ausubel et al., Short Protocols in Molecular Biology (1999) 4th Ed, John Wiley & Sons, Inc.) and chemical methods. The terms *capable of forming a hybrid* or *hybridizing sequence* in the context of the present specification relate to sequences that under the conditions existing within the cytosol of a mammalian cell, are able to bind selectively to their target sequence. Such hybridizing sequences may be contiguously reverse-complimentary to the target sequence, or may comprise gaps, mismatches or additional non-matching nucleotides. The minimal length for a sequence to be capable of forming a hybrid depends on its composition, with C or G nucleotides contributing more to the energy of binding than A or T/U nucleotides, and the backbone chemistry.

The term *Nucleotides* in the context of the present specification relates to nucleic acid or nucleic acid analogue building blocks, oligomers of which are capable of forming selective hybrids with RNA or DNA oligomers on the basis of base pairing. The term *nucleotides* in this context includes the classic ribonucleotide building blocks adenosine, guanosine, uridine (and ribosylthymine), cytidine, the classic deoxyribonucleotides deoxyadenosine, deoxyguanosine, thymidine, deoxyuridine and deoxycytidine. It further includes analogues of nucleic acids such as phosphotioates, 2'O-methylphosphothioates, *peptide nucleic acids* (PNA; N-(2-aminoethyl)-glycine units linked by peptide linkage, with the nucleobase attached to the alpha-carbon of the glycine) or *locked nucleic acids* (LNA; 2'O, 4'C methylene bridged RNA building blocks). Wherever reference is made herein to a *hybridizing sequence,* such hybridizing sequence may be composed of any of the above nucleotides, or mixtures thereof.

In the context of the present specification, the term *antibody* refers to whole antibodies including but not limited to immunoglobulin type G (IgG), type A (IgA), type D (IgD), type E (IgE) or type M (IgM), any antigen binding fragment or single chains thereof and related or derived constructs. A whole antibody is a glycoprotein comprising at least two heavy (H) chains and two light (L) chains inter-connected by disulfide bonds. Each heavy chain is comprised of a heavy chain variable region ($V_H$) and a heavy chain constant region ($C_H$). The heavy chain constant region is comprised of three domains, $C_H1$, $C_H2$ and $C_H3$. Each light chain is comprised of a light chain variable region (abbreviated herein as $V_L$) and a light chain constant region ($C_L$). The light chain constant region is comprised of one domain, $C_L$. The variable regions of the heavy and light chains contain a binding domain that interacts with an antigen. The constant regions of the antibodies may mediate the binding of the immunoglobulin to host tissues or factors, including various cells of the immune system (e.g., effector cells) and the first component of the classical complement system. Similarly, the term encompasses a so-called nanobody or single domain antibody, an antibody fragment consisting of a single monomeric variable antibody domain.

The term *antibody-like molecule* in the context of the present specification refers to a molecule capable of specific binding to another molecule or target with high affinity, particularly characterized by a Kd $\leq$ 10E-8 mol/l. An antibody-like molecule binds to its target similarly to the specific binding of an antibody. The term *antibody-like molecule* encompasses a repeat protein, such as a designed ankyrin repeat protein (Molecular Partners, Zürich), an engineered antibody mimetic proteins exhibiting highly specific and high-affinity target protein binding (see US2012142611, US2016250341, US2016075767 and US2015368302, all of which are incorporated herein by reference). The term antibody-like molecule further encompasses, but is not limited to, a polypeptide derived from armadillo repeat proteins, a polypeptide derived from leucine-rich repeat proteins and a polypeptide derived from tetratricopeptide repeat proteins.

The term *antibody-like molecule* further encompasses a specifically binding polypeptide derived from

- a protein A domain,
- fibronectin domain FN3,
- consensus fibronectin domains,
- a lipocalins (see Skerra, Biochim. Biophys. Acta 2000, 1482(1-2):337-50),
- a polypeptide derived from a Zinc finger protein (see Kwan et al. Structure 2003, 11(7):803-813),
- Src homology domain 2 (SH2) or Src homology domain 3 (SH3),
- a PDZ domain,
- gamma-crystallin,
- ubiquitin,
- a cysteine knot polypeptide or a knottin,

- cystatin,
- Sac7d,
- a triple helix coiled coil (also known as alphabody),
- a Kunitz domain or a Kunitz-type protease inhibitor and
- a carbohydrate binding module 32-2.

In the context of the present specification, the term *humanized antibody* refers to an antibody originally produced by immune cells of a non-human species, the protein sequences of which have been modified to increase their similarity to antibody variants produced naturally in humans. The term *humanized antibody* as used herein includes antibodies in which CDR sequences derived from the germline of another mammalian species, such as a mouse, have been grafted onto human framework sequences. Additional framework region modifications may be made within the human framework sequences as well as within the CDR sequences derived from the germline of another mammalian species.

The term *specific binding* in the context of the present invention refers to a property of ligands that bind to their target with a certain affinity and target specificity. The affinity of such a ligand is indicated by the dissociation constant of the ligand. A specifically reactive ligand has a dissociation constant of $\leq 10^{-7}$ mol/L when binding to its target, but a dissociation constant at least three orders of magnitude higher in its interaction with a molecule having a globally similar chemical composition as the target, but a different three-dimensional structure.

In the context of the present specification, the term *dissociation constant (KD)* is used in its meaning known in the art of chemistry and physics; it refers to an equilibrium constant that measures the propensity of a complex composed of [mostly two] different components to dissociate reversibly into its constituent components. The complex can be e.g. an antibody-antigen complex AbAg composed of antibody Ab and antigen Ag. $K_D$ is expressed in molar concentration [mol/l] and corresponds to the concentration of [Ab] at which half of the binding sites of [Ag] are occupied, in other words, the concentration of unbound [Ab] equals the concentration of the [AbAg] complex. The dissociation constant can be calculated according to the following formula:

$$K_D = \frac{[Ab] * [Ag]}{[AbAg]}$$

*[Ab]: concentration of antibody; [Ag]: concentration of antigen; [AbAg]: concentration of antibodyantigen complex*

In the context of the present specification, the terms *off-rate* (Koff;[1/sec]) and *on-rate* (Kon; [1/sec*M]) are used in their meaning known in the art of chemistry and physics; they refer to a rate constant that measures the dissociation (Koff) or association (Kon) of 5 an antibody with its target antigen. Koff and Kon can be experimentally determined using methods well established in the art. A method for determining the Koff and Kon of an antibody employs surface plasmon resonance. This is the principle behind biosensor systems such as the Biacore® or the ProteOn® system. They can also be used to determine the dissociation constant KD by using the following formula:

$$K_D = \frac{[K_{off}]}{[K_{on}]}$$

The term gene refers to a polynucleotide containing at least one open reading frame (ORF) that is capable of encoding a particular polypeptide or protein after being transcribed and translated. A polynucleotide sequence can be used to identify larger fragments or full-length coding sequences of the gene with which they are associated. Methods of isolating larger fragment sequences are known to those of skill in the art.

The terms *gene expression* or alternatively *gene product* refer to the processes - and products thereof - of nucleic acids (RNA) or amino acids (e.g., peptide or polypeptide) being generated when a gene is transcribed and translated.

As used herein, *expression* refers to the process by which DNA is transcribed into mRNA and/or the process by which the transcribed mRNA is subsequently translated into peptides, polypeptides or proteins. If the polynucleotide is derived from genomic DNA, expression may include splicing of the mRNA in a eukaryotic cell.

The term *antisense oligonucleotide* in the context of the present specification relates to an oligonucleotide having a sequence substantially complimentary to, and capable of hybridizing to, an RNA. Antisense action on such RNA will lead to modulation, particular inhibition or suppression of the RNA's biological effect. If the RNA is an mRNA, expression of the resulting gene product is inhibited or suppressed. Antisense oligonucleotides can consist of DNA, RNA, nucleotide analogues and/or mixtures thereof. The skilled person is aware of a variety of commercial and non-commercial sources for computation of a theoretically optimal antisense sequence to a given target. Optimization can be performed both in terms of nucleobase sequence and in terms of backbone (ribo, deoxyribo, analogue) composition. Many sources exist for delivery of the actual physical oligonucleotide, which generally is synthesized by solid state synthesis.

The term *siRNA* (small/short interfering RNA) in the context of the present specification relates to an RNA molecule capable of interfering with the expression (in other words: inhibiting or preventing the expression) of a gene comprising a nucleic acid sequence complementary or hybridizing to the sequence of the siRNA in a process termed RNA interference. The term *siRNA* is meant to encompass both single stranded siRNA and double stranded siRNA. siRNA is usually characterized by a length of 17-24 nucleotides. Double stranded siRNA can be derived from longer double stranded RNA molecules (dsRNA). According to prevailing theory, the longer dsRNA is cleaved by an endo-ribonuclease (called Dicer) to form double stranded siRNA. In a nucleoprotein complex (called RISC), the double stranded siRNA is unwound to form single stranded siRNA. RNA interference often works via binding of an siRNA molecule to the mRNA molecule having a complementary sequence, resulting in degradation of the mRNA. RNA interference is also possible by binding of an siRNA molecule to an intronic sequence of a pre-mRNA (an immature, non-spliced mRNA) within the nucleus of a cell, resulting in degradation of the pre-mRNA.

The term *shRNA* (small hairpin RNA) in the context of the present specification relates to an artificial RNA molecule with a tight hairpin turn that can be used to silence target gene expression via RNA interference (RNAi).

The term *sgRNA* (single guide RNA) in the context of the present specification relates to an RNA molecule capable of sequence-specific repression of gene expression via the CRISPR (clustered regularly interspaced short palindromic repeats) mechanism.

The term *miRNA* (microRNA) in the context of the present specification relates to a small non-coding RNA molecule (containing about 22 nucleotides) that functions in RNA silencing and post-transcriptional regulation of gene expression.

The term *detectable CTC-neutrophil cluster* in the context of the present specification relates to the detection of CTC-neutrophil clusters in the bloodstream as described in CTC capture in the methods section.

As used herein, the term *treating* or *treatment* of any disease or disorder (e.g. cancer) refers in one embodiment, to ameliorating the disease or disorder (e.g. slowing or arresting or reducing the development of the disease or at least one of the clinical symptoms thereof). In another embodiment "treating" or "treatment" refers to alleviating or ameliorating at least one physical parameter including those which may not be discernible by the patient. In yet another embodiment, "treating" or "treatment" refers to modulating the disease or disorder, either physically, (e.g., stabilization of a discernible symptom), physiologically, (e.g., stabilization of a physical parameter), or both. Methods for assessing treatment and/or prevention of disease are generally known in the art, unless specifically described hereinbelow.

Dosage forms may be for enteral administration, such as nasal, buccal, rectal, transdermal or oral administration, or as an inhalation form or suppository. Alternatively, parenteral administration may be used, such as subcutaneous, intravenous, intrahepatic or intramuscular injection forms. Optionally, a pharmaceutically acceptable carrier and/or excipient may be present.

Topical administration is also within the scope of the advantageous uses of the invention. The skilled artisan is aware of a broad range of possible recipes for providing topical formulations, as exemplified by the content of Benson and Watkinson (Eds.), Topical and Transdermal Drug Delivery: Principles and Practice (1st Edition, Wiley 2011, ISBN-13: 978-0470450291); and Guy and Handcraft: Transdermal Drug Delivery Systems: Revised and Expanded (2nd Ed., CRC Press 2002, ISBN-13: 978-0824708610); Osborne and Amann (Eds.): Topical Drug Delivery Formulations (1st Ed. CRC Press 1989; ISBN-13: 978-0824781835).

*Detailed description of the invention*

A first aspect of the invention relates to a non-agonist ligand specifically binding to a target protein implicated in promoting metastasis by aiding the association of circulating tumour cells with leukocytes. The target protein is selected from the following list:

- VCAM1 (CD106) (Entrez Gene-ID: 7412, Uniprot: P19320),

- CD49d (Entrez Gene-ID: 3676, Uniprot: P13612),

- CD29 (Entrez Gene-ID: 3688, Uniprot: P05556),

- IL6 receptor (CD126) (Entrez Gene-ID: 3570, Uniprot: P08887),

- IL6 (Entrez Gene-ID: 3569, Uniprot: P05231),

- IL1β receptor (IL1R1) (Entrez Gene-ID: 3554, Uniprot: P14778),

- IL1β (Entrez Gene-ID: 3553, Uniprot: P01584),

- F11R (Entrez Gene-ID: 50848, Uniprot: Q9Y624),

- ICAM1 (Entrez Gene-ID: 3383, Uniprot: P05362), or

- ITGB2 (Entrez Gene-ID: 3689, Uniprot: P05107),

The non-agonist ligand specifically reactive to any one of the above target proteins is provided for use in treatment or prevention of metastatic cancer.

Modulation of VCAM1 activity is demonstrated by the results shown herein to lead to abrogation of CTC-neutrophil cluster formation and subsequent impairment of metastasis.

Via the same mechanism, the modulation of the VCAM1 receptor, composed of CD49d and CD29, will lead to abrogation of CTC-neutrophil cluster formation.

The results also demonstrate that F11R, ICAM1, and ITGB2 are involved in CTC-neutrophil cluster formation.

The inventors found that IL1β and IL6 are among the most frequently expressed cytokines in CTC-associated neutrophils. The inventors observed that treatment with IL6 or IL1β leads to a faster metastasis development and shorter overall survival of mice, while knockout of IL6 or IL1β receptors on cancer cells suppressed their proliferative advantage. Thus, both the downregulation of IL6 or IL1β and of their receptors is a promising approach in treatment of metastasis.

In certain embodiments, the metastatic cancer is metastatic breast cancer. While the data given herein mainly derive from clinical samples and models of breast cancer, the mechanisms underlying metastasis will be applicable to metastasis of other epithelial cancers such as prostate cancer, colon cancer, lung cancer and cancer cells of other origin.

In certain embodiments, the non-agonist ligand is an antibody, an antibody fragment, an aptamer or an antibody-like molecule.

In certain embodiments, the non-agonist ligand is a human or a humanized antibody.

In certain embodiments, the ligand is canakinumab (CAS No. 914613-48-2). Canakinumab is a monoclonal antibody targeting interleukin-1 beta. It was developed by Novartis and is marketed under the tradename Ilaris. The drug is provided as lyophilized powder for preparation of an infusion solution.

In certain embodiments, the ligand is tocilizumab (CAS No. 375823-41-9), a humanized monoclonal antibody against the interleukin-6 receptor. The drug is marketed as Actemra or RoActemra by Roche. It is provided as a solution for injection.

In certain embodiments, the ligand is siltuximab (CAS No. 541502-14-1). Siltuximab is a chimeric (human/murine) antibody reactive to interleukin-6. It is approved for human use and marketed under the trademark Sylvant as lyophilized powder for preparation of an infusion solution by Janssen-Cilag.

In certain embodiments, a metastatic cancer cells' capability of extravasation, infiltration into stroma of organs, survival or growth at distant sites is diminished by the ligand. Thereby, the metastatic potential of the cancer cells is reduced.

An alternative of this aspect of the invention relates to a nucleic acid molecule encoding the ligand according to the first aspect for use in treatment or prevention of metastatic cancer. While the administration of commercially available, approved antibody drugs may be the fastest way to deploy the current invention in humans, expressing a therapeutic protein from DNA or RNA in the patient has long been a promise to circumvent the cost and regulatory burden of novel antibody development and may well prove to be a viable alternative to applying the teaching of the invention in human patients.

A second aspect of the invention relates to a nucleic acid molecule comprising, or consisting of, an inhibitor nucleic acid sequence capable of directly or indirectly downregulating or inhibiting expression of a target nucleic acid sequence encoding a protein selected from:

- VCAM1 (CD106),

- CD49d,

- CD29,

- IL6 receptor (CD126),

- IL6,

- IL1β receptor (IL1R1),

- IL1β

- F11R,

- ICAM1 and

- ITGB2,

for use in treatment or prevention of metastatic cancer.

In general, both antisense targeting of the gene target implied in formation of CTC-neutrophil clusters and promotion of metastasis, and a CRISPR or analogous approach is contemplated.

In certain embodiments, said inhibitor nucleic acid sequence is able to specifically hybridize with a sequence or subsequence of

- an exon comprised in said target nucleic acid sequence,
- an intron comprised in said target nucleic acid sequence,
- a promoter region modulating expression of said target nucleic acid sequence, and/or
- an auxiliary sequence regulating expression of said target nucleic acid sequence.

Hybridization of the inhibitor nucleic acid sequence with the exon, intron, promoter or auxiliary sequence of the target nucleic acid sequence as described above leads to a decreased or inhibited transcription or translation of the target nucleic acid sequence. The mechanism employed may be degradation of mRNA, e.g. by RNA interference, CRISPR/Cas system, inhibition of translation or blockage of a promoter or enhancer region.

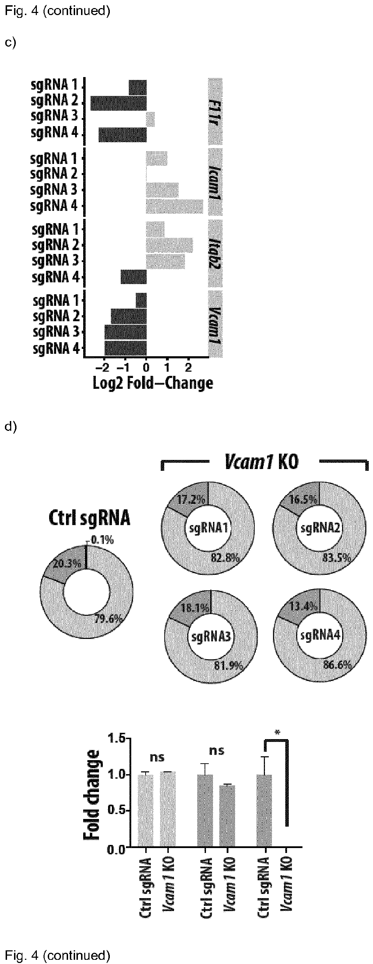

Fig. 4d and the examples show the use of CRISPR to knockout VCAM1. From a practical standpoint (e.g. in patients), certain hurdles will need to be taken until the targeted editing of genomic DNA is a feasible clinical application, but current developing efforts to turn CRISPR editing "packages" (Cas9 or equivalent plus sgRNA) deliverable to particular organs make this a prospect with hopes for realisation within the lifetime of the current application

siRNA or shRNA, delivered to the tumour through a particular carrier systems (liposomes etc.) are an even more immediate application.

In certain embodiments, the auxiliary sequence is an enhancer sequence. The enhancer sequence is a short (50-1500 bp) region of DNA that can be bound by activators to increase the likelihood that transcription of the target nucleic acid sequence will occur. The inhibitor nucleic acid sequence will decrease the activity of the enhancer sequence.

In certain embodiments, the auxiliary sequence is a long non-coding RNA sequence. Long non-coding RNAs are transcripts longer than 200 nucleotides that are not translated into protein, but regulate transcription or translation of the target nucleic acid sequence.

In certain embodiments, said inhibitor nucleic acid sequence is an antisense oligonucleotide. In certain embodiments, said inhibitor nucleic acid sequence is an siRNA. In certain embodiments, said inhibitor nucleic acid sequence is an shRNA. In certain embodiments, said inhibitor nucleic acid sequence is an sgRNA. In certain embodiments, said inhibitor nucleic acid sequence is an miRNA.

In certain embodiments, the inhibitor nucleic acid sequence comprises or consists of nucleoside analogues.

A third aspect of the invention relates to a method for assigning a likelihood of developing metastasis to a patient, wherein a high likelihood is assigned if

- said patient has at least one detectable CTC-neutrophil cluster in the bloodstream; and/or
- a tumor of said patient has a mutation in gene *TLE1* selected from 1787G>A or 1509G>C; and/or
- a tumor of said patient has a mutation in genes belonging to the frequently mutated gene list of table 3 in patients with CTC-neutrophil clusters and /or
- more than 40%, particularly 50%, more particularly 60% of mutations inside a tumor of said patient are C>T substitutions.

A high likelihood of developing metastasis is assigned to a patient if one, particularly two, more particularly three, most particularly all four criteria above are fulfilled.

The likelihood of detecting at least one CTC-neutrophil cluster in the bloodstream changes with the quantity of volume; the skilled person understands that the volume of analysed blood depends on the inherent limits of a given CTC isolation technology. Importantly, the detection of even one CTC-neutrophil cluster changes the prognosis so dramatically that the variation of this change as a function of sample volume is negligible.

The skilled person is aware of methods for detecting mutations. Methods include several types of PCR (polymerase chain reaction), RFLP (Restriction Fragment Length Polymorphism), and sequencing.

A fourth aspect of the invention relates to the use of the non-agonist ligand or the nucleic acid molecule according to the invention in treatment or prevention of metastatic cancer, wherein a high likelihood of developing metastasis is assigned to the patient according as provided herein.

Similarly within the scope of the present invention is a method or treating metastatic cancer in a patient in need thereof, comprising administering to the patient a non-agonist ligand or antisense molecule according to the above description.

Similarly, a dosage form for the prevention or treatment of metastatic cancer is provided, comprising a non-agonist ligand or antisense molecule according to any of the above aspects or embodiments of the invention.

Dosage forms may be for enteral administration, such as nasal, buccal, rectal, transdermal or oral administration, or as an inhalation form or suppository. Alternatively, parenteral administration may be used, such as subcutaneous, intravenous, intrahepatic or intramuscular injection forms. Optionally, a pharmaceutically acceptable carrier and/or excipient may be present.

Wherever alternatives for single separable features such as, for example, a non-agonist ligand or a diagnostic method are laid out herein as "embodiments", it is to be understood that such alternatives may be combined freely to form discrete embodiments of the invention disclosed herein.

The invention is further illustrated by the following examples and figures, from which further embodiments and advantages can be drawn. These examples are meant to illustrate the invention but not to limit its scope.

*Brief description of the figures*

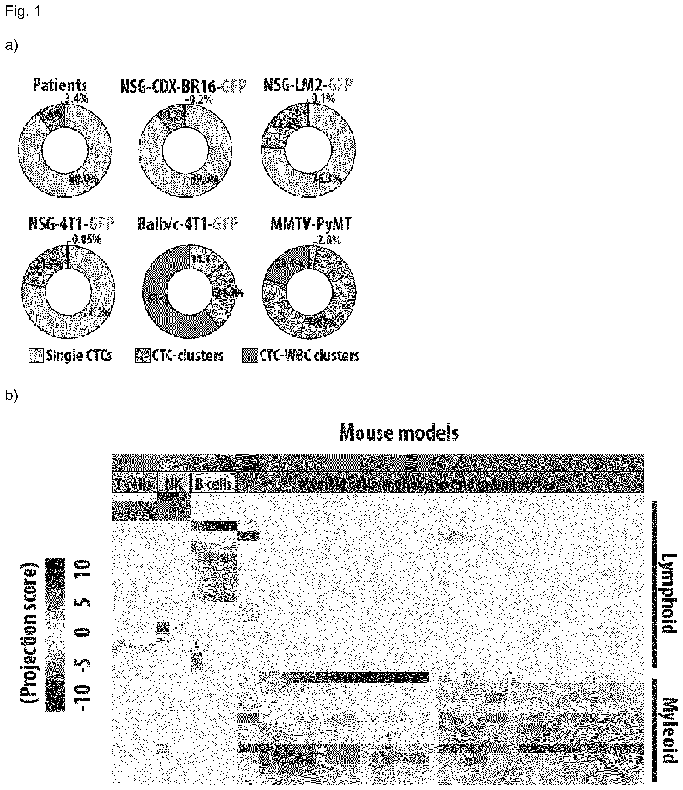

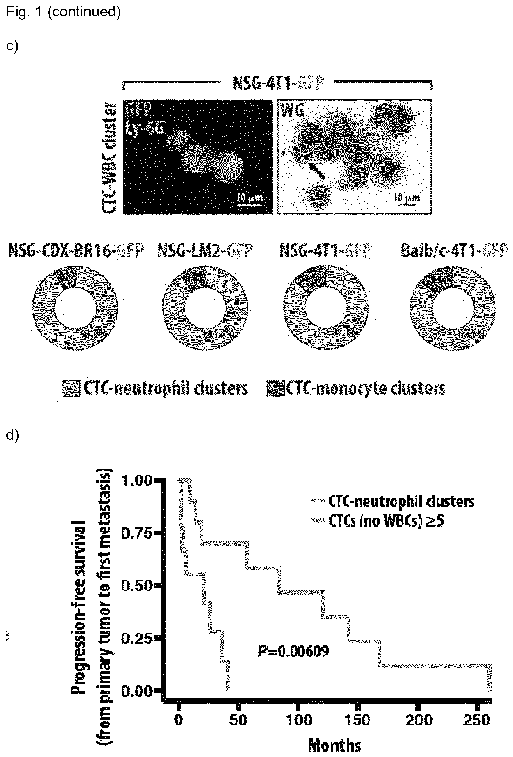

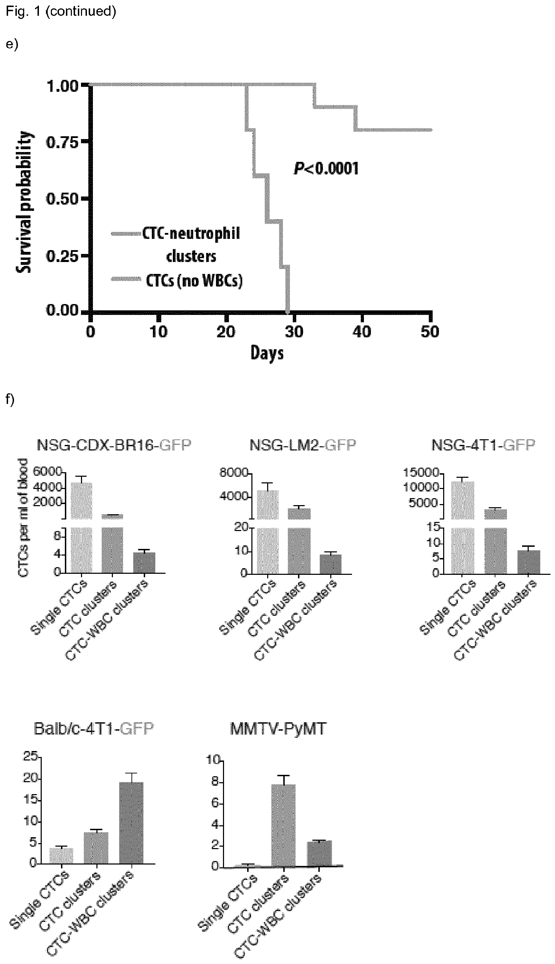

Fig. 1 **CTC-neutrophil clusters are highly efficient metastatic precursors. a,** Pie charts displaying the mean percentage of single CTCs (*grey*), CTC clusters (*green*) and CTC-WBC clusters (*red*) in breast cancer patients and mouse models. *n*=34 for patients; *n*=8 for NSG-CDX-BR16-*GFP*, NSG-LM2-*GFP* and NSG-4T1-*GFP*; *n*=7 for Balb/c-4T1-*GFP*; *n*=5 for MMTV-PyMT. b, RCA clustering of CTC-associated WBCs and reference WBCs from mouse models. **c,** Representative images of CTC-WBC clusters stained for Ly-6G (neutrophils; *gold*) together with GFP imaging (cancer cells; *green*) (*left*) or processed with the Wright-Giemsa (WG) assay to define nuclear morphology (*right*). The pie charts show the mean percentage of CTC-neutrophil clusters and CTC-monocyte clusters in individual models (*bottom*). **d,** Kaplan-Meier plot showing progression-free survival of breast cancer patients with ≥ 1 CTC-neutrophil cluster versus patients with ≥ 5 CTC per 7.5ml of peripheral blood. *n*=9 for CTC-neutrophil clusters and *n*=10 for ≥ 5 CTCs; *P*= 0.00609 by pairwise Log-Rank test. **e,** Kaplan-Meier plot showing overall survival of mice. *n*=5 for CTC-neutrophil clusters and *n*=10 for CTCs alone; *P*< 0.0001 by pairwise Log-Rank test. **f,** Bar graphs showing the mean counts of single CTCs, CTC clusters and CTC-WBC clusters in five different breast cancer mouse models. Error bars represent S.E.M.; *n*=8 for NSG-CDX-BR16-*GFP*, NSG-LM2-*GFP*, NSG-4T1-*GFP*, *n*=7 for Balb/c-4T1-*GFP*, *n*=5 for MMTV-PyMT.

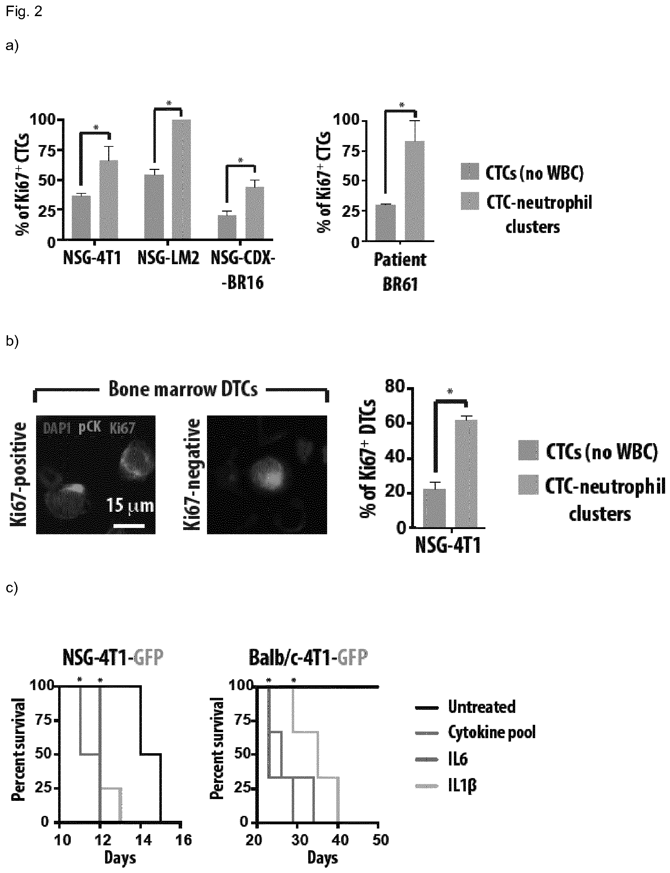

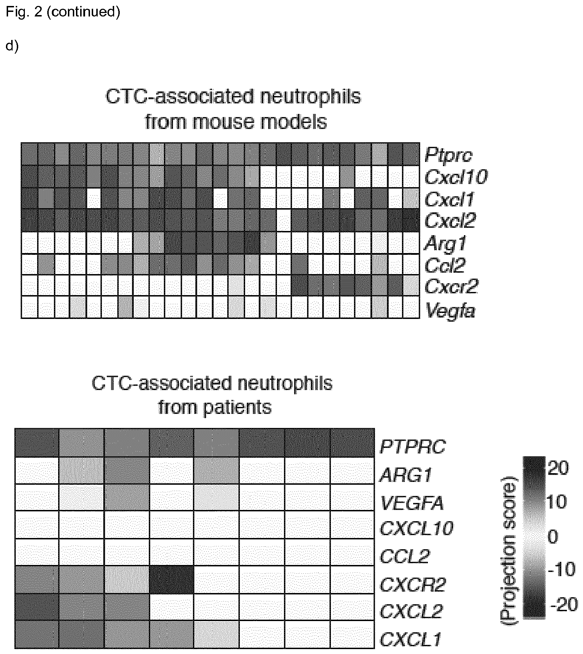

Fig. 2 **CTCs from CTC-neutrophil clusters activate a gene expression program of cell cycle progression. a,** The bar graphs show the mean percent of Ki67-positive CTCs. *n*=3 for all; error bars represent S.E.M.; *P*< 0.03 by Student's *t* test. **b,** Representative pictures of DTCs stained for pan-cytokeratin (pCK; *green*), Ki67 (*purple*) and DAPI (nuclei; *blue*) (*left*). The bar graph shows the mean percent of Ki67-positive DTCs (*right*). *n*=5 for CTC-neutrophil clusters and *n*=3 for CTCs alone; **P*< 0.002 by Student's *t* test. **c,** Kaplan-Meier survival analysis of NSG (*left*) or Balb/c (*right*) mice injected with cytokine-treated 4T1-*GFP* cells. *n*=4 for NSG-4T1-*GFP* and *n*=3 for Balb/c-4T1-*GFP*; *P*=<0.03 by log-rank Mantel-Cox test. **d,** Heatmap showing the projection scores of mouse- (*top*) and patient-derived (*bottom*) CTC-associated neutrophils (*columns*) on pro-tumoral (N2) neutrophil markers (*rows*).

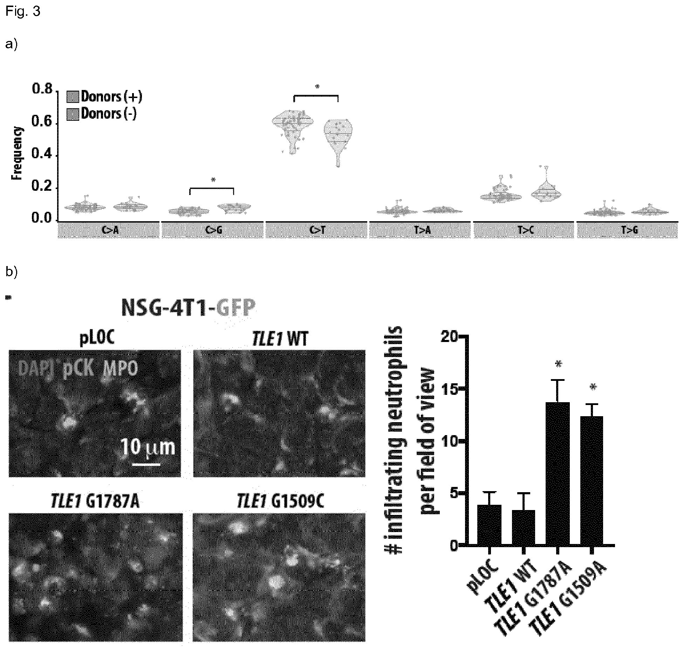

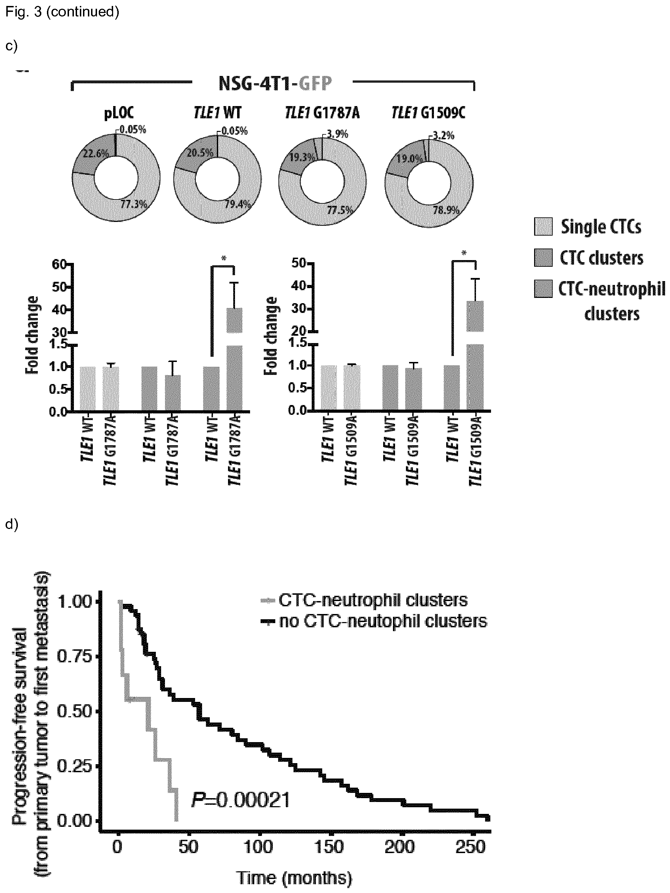

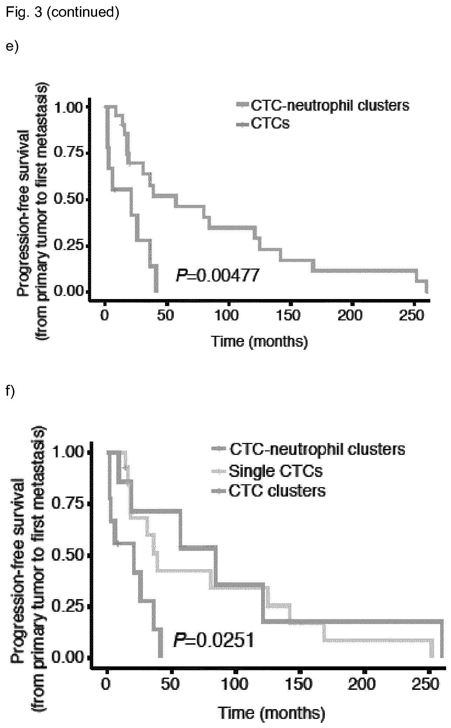

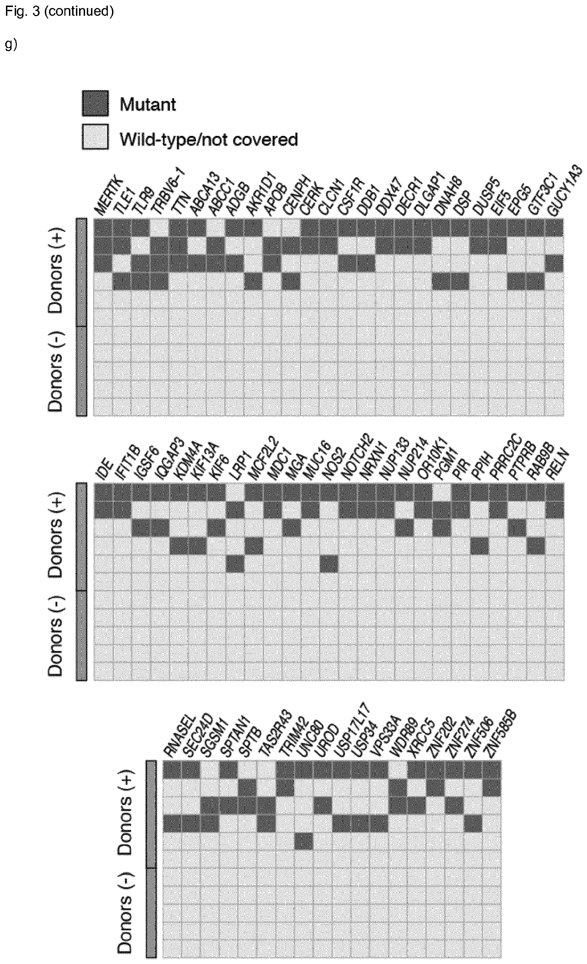

Fig. 3 **Whole-exome sequencing highlights recurrent mutational events in CTCs from CTC-neutrophil clusters. a,** Nucleotide substitution pattern among putative somatic mutations in CTCs isolated from donors with CTC-neutrophil clusters (Donors (+)) versus donors without CTC-neutrophil clusters (Donors (-)). *n*=6 for Donors (+) and *n*=5 for Donors (-); **P*<0.05 by Wilcoxon sign-ranked test. **b,** Representative images of the primary tumor of mice that were injected with either 4T1-pLOC, 4T1-*TLE1* wild type (WT), 4T1-*TLE1* G1787A or 4T1-*TLE1* G1509C cells and stained for pan-cytokeratin (pCK; *green*), myeloperoxidase (MPO; *gold*) and DAPI (nuclei; *blue*) (*top*). The bar graph shows the mean number of neutrophils per field of view (*bottom*). *n*=3; error bars represent S.E.M.; **P*< 0.002 by Student's *t* test. **c,** Pie charts displaying the mean percentage of single CTCs *(grey),* CTC clusters *(green)* and CTC-neutrophil clusters *(gold)* in mice injected with 4T1-pLOC, 4T1-*TLE1* WT, 4T1-*TLE1* G1787A or 4T1-*TLE1* G1509C cells (*top*). *n*=3 for all groups. The bar graph shows the mean fold change of CTC ratios (*bottom*). *n*=3; error bars represent S.E.M.; **P*< 0.03 by Student's *t* test.

**d**, Kaplan-Meier progression-free survival (PFS) analysis comparing patients with at least 1 CTC-neutrophil cluster per 7.5ml of peripheral blood versus patients with no CTC-neutrophil cluster per 7.5ml of peripheral blood (i.e., both positive and negative for CTCs). $n$=48 for no CTC-neutrophil clusters and $n$=9 for CTC-neutrophil clusters; $P$=0.00021 by pairwise Log-Rank test. **e**, Kaplan-Meier PFS analysis comparing patients with at least 1 CTC-neutrophil cluster per 7.5ml of peripheral blood versus patients with at least 1 CTC per 7.5ml of peripheral blood but without CTC-neutrophil clusters. $n$=21 for CTCs and $n$=9 for CTC-neutrophil clusters; $P$=0.00477 by pairwise Log-Rank test. **f**, Kaplan-Meier PFS analysis comparing patients with at least 1 CTC-neutrophil cluster per 7.5ml of peripheral blood, patients with at least 1 single CTC per 7.5ml of peripheral blood but without CTC-neutrophil clusters, and patients with at least 1 CTC cluster per 7.5ml of peripheral blood but without CTC-neutrophil clusters. $n$=14 for single CTCs, $n$=7 for CTC-clusters and $n$=9 for CTC-neutrophil clusters; $P$=0.0251 by pairwise Log-Rank test. Of note, these results are consistent with our previous observations whereby differences in progression-free survival between breast cancer patients with single CTCs or CTC-clusters were visible only when CTC clusters were present for multiple time points along disease progression. In the case of CTC-neutrophil clusters instead, their presence during one single time point is already predictive of a poor prognosis. **g**, Mutational landscape specific to donors with CTC-neutrophil clusters (Donors (+)). The tile plot represent genes (columns) containing predicted high-impact mutations (see Methods) in at least 2 donors with CTC-neutrophil clusters (Donors (+)) and in none of the donors without CTC-neutrophil clusters (Donors(-)).

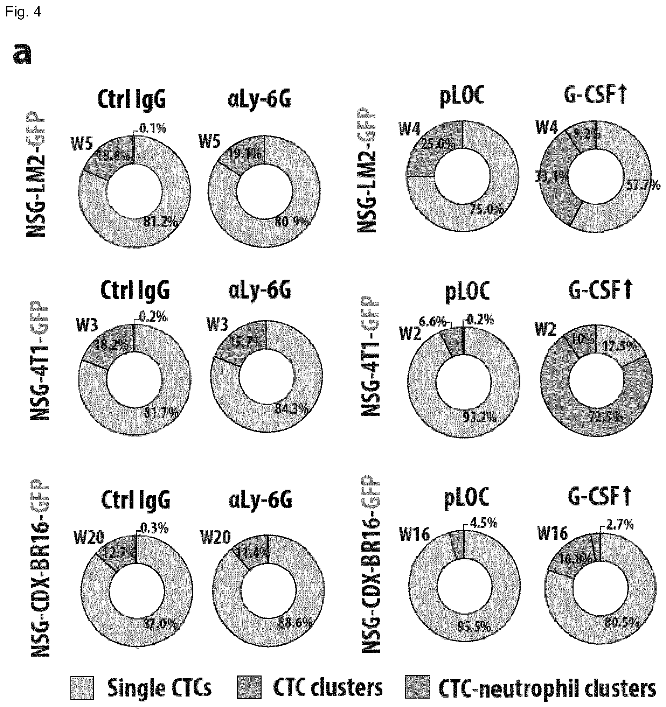

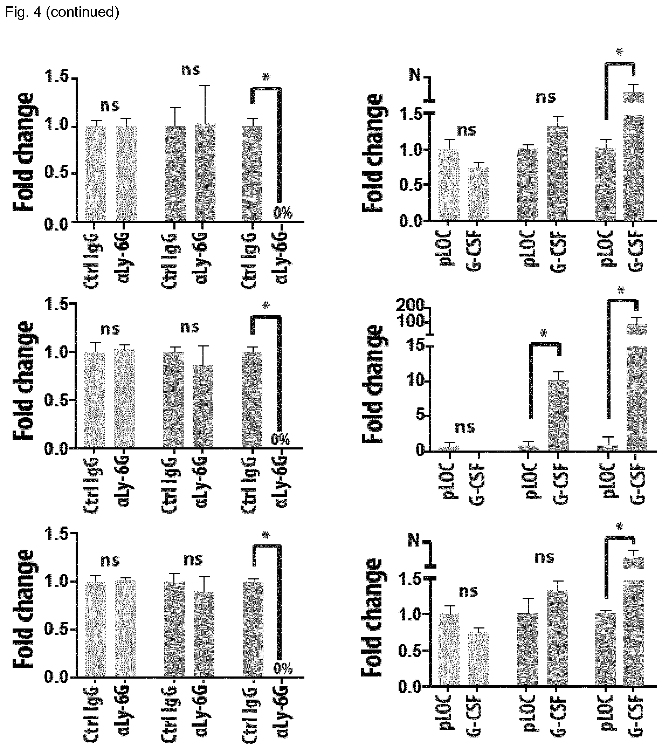

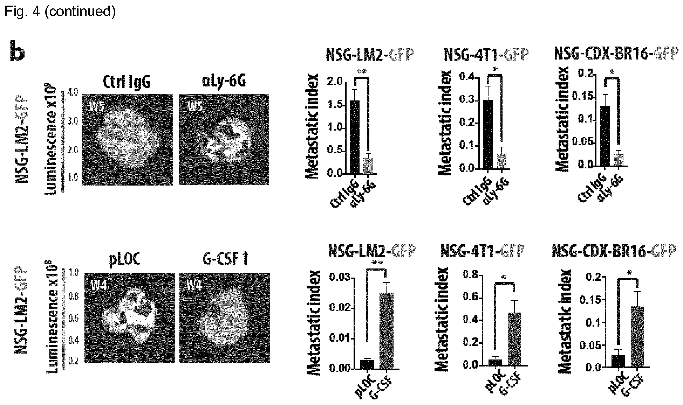

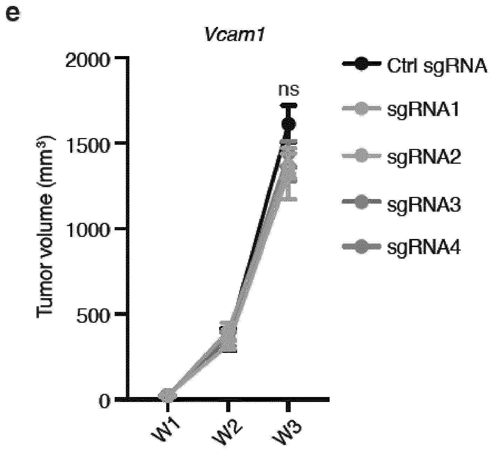

Fig. 4   **Identification of vulnerabilities of CTC-neutrophil clusters. a,** Pie charts displaying the mean percentage of single CTCs (*grey*), CTC clusters (*green*) and CTC-neutrophil clusters *(gold)* in mice treated with αLy-6G antibodies or G-CSF overexpression (*top*). W = weeks upon tumor development; $n$=3-6. The bar graphs show the mean fold change of CTC ratios from mice treated with αLy-6G antibodies or G-CSF overexpression (*bottom*). $n$=3-6; error bars represent S.E.M.; *P<0.04 by Student's $t$ test. **b**, Representative bioluminescence images of lungs from mice treated with αLy-6G antibodies or G-CSF overexpression (*left*). W = weeks upon tumor development; $n$=3-6. The bar graphs show the mean metastatic index of mice treated with αLy-6G antibodies or G-CSF overexpression (*right*). $n$=3-6; error bars represent S.E.M.; *P<0.03 **P<0.01 by Student's $t$ test. **c**, Bar plot showing the log2 fold change (FC) of individual sgRNAs found in CTCs from CTC-neutrophil clusters versus CTCs alone. sgRNAs whose representation was reduced in CTCs from CTC-neutrophil clusters are shown in *dark red*. $n$=3. **d**, Pie charts displaying the mean percentage of single CTCs *(grey),* CTC clusters *(green)* and CTC-neutrophil clusters *(gold)* in NSG mice carrying 4T1-Cas9-*GFP* tumors expressing a control sgRNA (Ctrl sgRNA) or individual sgRNAs targeting *Vcam1* (*Vcam1* KO) (*top*); $n$=4. The bar graph shows the mean fold change of CTC ratios from mice carrying 4T1-Cas9-*GFP* tumors expressing a control sgRNA (Ctrl sgRNA) or individual sgRNAs targeting *Vcam1* (*Vcam1* KO) (*bottom*); $n$=4; error bars represent S.E.M.; *P<0.02 by Student's $t$ test. **e**, Tumor growth curves of mice injected with 4T1-Cas9-*GFP* cells expressing a control vector (CTRL sgRNA) or individual sgRNAs targeting *Vcam1.* $n$=4; error bars represent S.E.M.; ns= not significant.

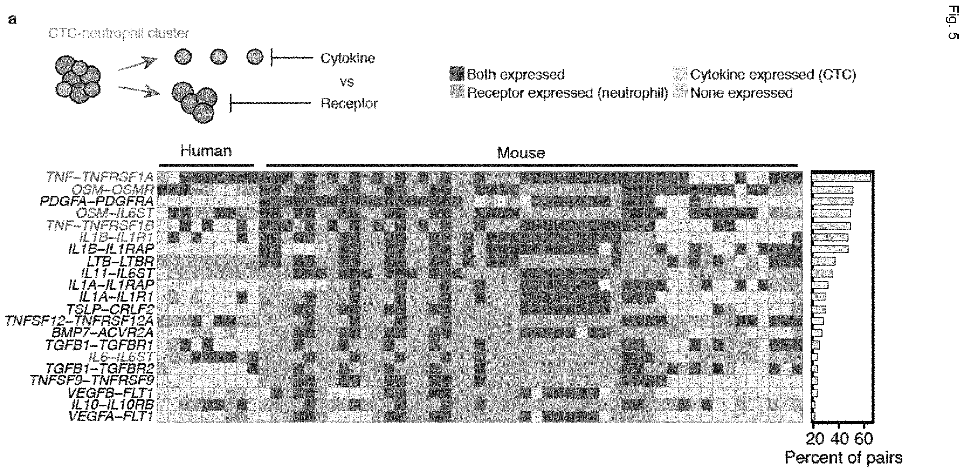

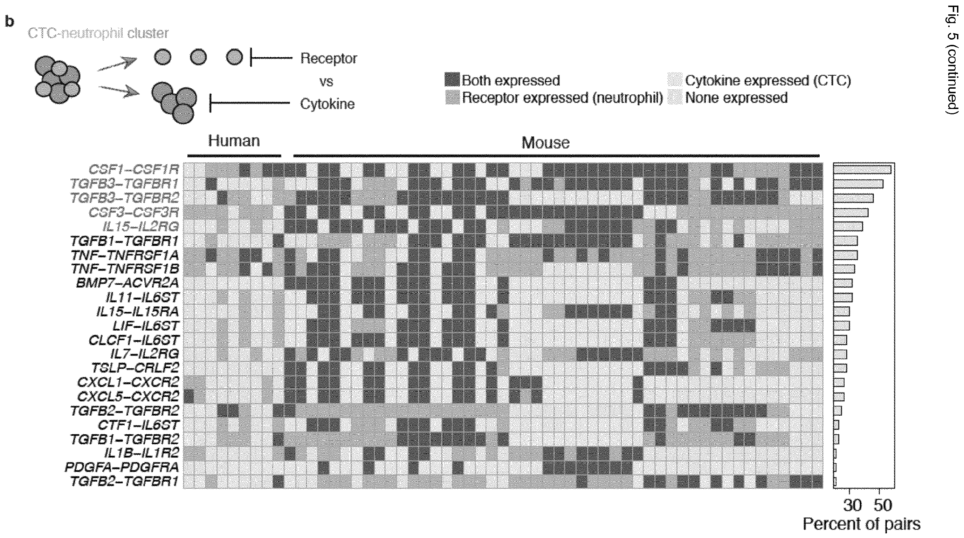

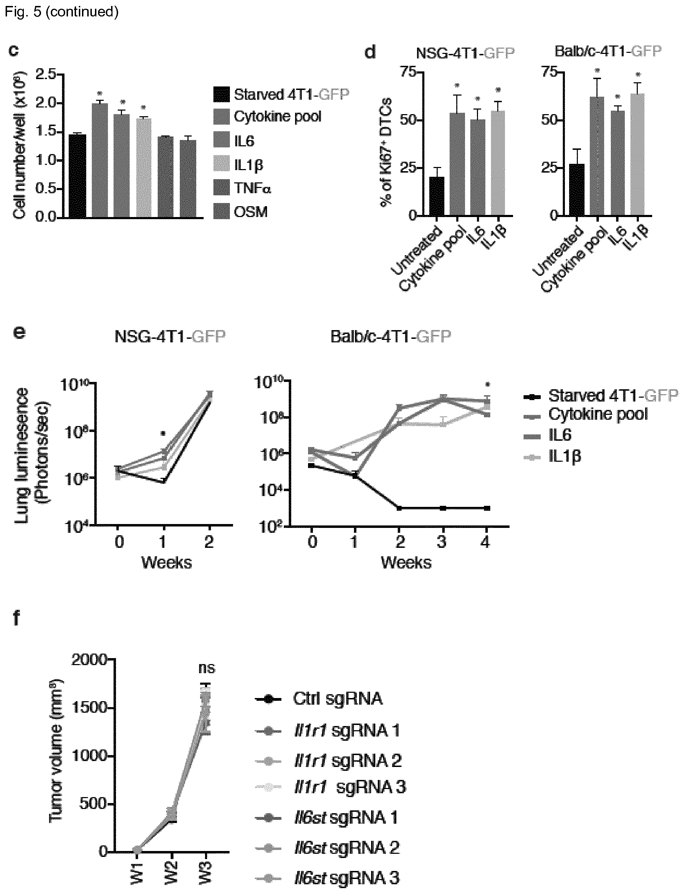

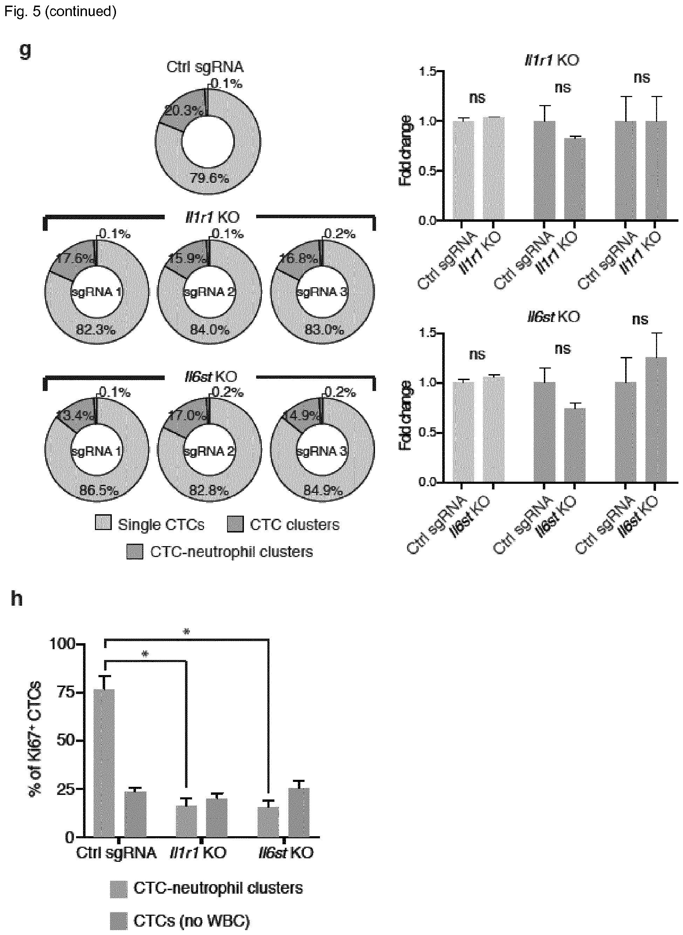

Fig. 5   **Characterization of cytokine-mediated crosstalk between CTCs and CTC-associated neutrophils. a**, Schematic of the experimental design. Expression levels of all known cytokine receptors in CTCs from CTC-neutrophil clusters are compared to the expression levels of all known cytokines from the corresponding neutrophils (*top*). The heatmap shows the transcriptional landscape of cytokines and their receptors that are expressed (log2 CPM $\geq$ 5) in at least 20% of the CTC-neutrophil clusters (*bottom*). The cytokine-receptor pairs that are most frequently expressed in human cells (either from patients or patient-derived models) are shown in *red.* **b**, Schematic of the experimental design. Expression levels of all known cytokines in CTCs from CTC-neutrophil clusters are compared to the expression levels of all known cytokine receptors from the corresponding neutrophils (*top*). The heatmap represents the expression landscape of cytokines and their receptors that are expressed (CPM $\geq$ 5) in at least 20% of the CTC-neutrophil clusters (*bottom*). The cytokine-receptor pairs that are expressed in at least 40% of CTC-neutrophil clusters (from both human and mouse cells) are shown in *red.* **c**, The bar graph shows the mean 4T1-*GFP* cell number upon starvation and stimulation with IL6, IL1β, TNFα, OSM or all four cytokines together (cytokine pool). $n$=3; error bars represent S.E.M.; *P< 0.05 by Student's $t$ test. **d**, Bar graphs showing the mean percentage of Ki67-positive disseminated tumor cells (DTCs) from bone marrow of mice injected with cytokine-treated 4T1-*GFP* cells. DTCs were harvested at the time of overt metastatic disease for both models. $n$=3 for all; error bars represent S.E.M,; *P=<0.05 by Student's $t$ test. **e**, Plots show normalized bioluminescence signal from the lungs of mice upon injection of *4T1-GFP* cells (untreated or cytokine-treated). $n$=4 for all; error bars represent S.E.M,; *P=<0.05 by Student's $t$ test. **f,** Tumor growth curves of NSG mice injected with 4T1-Cas9-*GFP* cells expressing a control vector (Ctrl sgRNA) or sgRNAs targeting *Ilr1* or *Il6st*. $n$=3; error bars represent S.E.M.; ns= not significant. **g,** Pie charts displaying the mean percentage of single CTCs *(grey)*, CTC clusters (*green*) and CTC-neutrophil clusters (*gold*) in NSG mice carrying 4T1-Cas9-*GFP* tumors expressing a control sgRNA (Ctrl sgRNA) or individual sgRNAs targeting *1l1r1* (*Il1r1* KO) or *Il6st* (*Il6st* KO) (*left*); $n$=3. The bar graphs show the mean fold change of CTC ratios from NSG mice carrying 4T1-Cas9-*GFP* tumors expressing a control sgRNA (Ctrl sgRNA) or individual sgRNAs targeting *Il1r1* (*Il1r1* KO) or *Il6st* (*Il6st* KO) (*right*); *n*=3; error bars represent S.E.M.; ns= not significant. **h,** The bar graph shows the mean percent of Ki67-positive CTCs in NSG mice carrying 4T1-Cas9-GFP tumors expressing a control sgRNA (Ctrl sgRNA) or individual sgRNAs targeting *Il1r1* (*Il1r1* KO) or *Il6st* (*Il6st* KO). *n*=3; error bars represent S.E.M.; *P< 0.02 by Student's *t* test

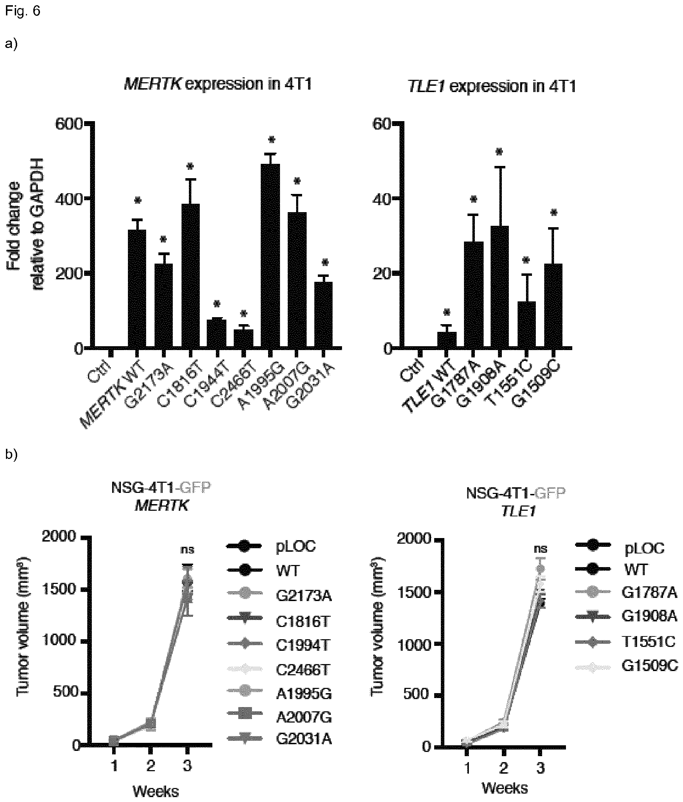

Fig. 6     **a,** *MERTK* and *TLE1* WT or mutated genes were lentivirally transduced in 4T1 cells and their expression was quantified by real-time PCR. The bar graphs show the mean fold change of RNA expression compared to untransduced (Ctrl) cells. *n*=3; error bars represent S.E.M.; *P=0.004 by Student's *t* test. **b,** Plots show tumor growth over time in NSG mice injected with 4T1 cells carrying an empty vector (pLOC), WT or mutated *MERTK* (*left*) or *TLE1* (*right*). *n*=3; error bars represent S.E.M.

Examples

*Example 1:*

The inventors first sought to determine the number and composition of CTC-WBC clusters in breast cancer patients and mouse models. The inventors obtained blood samples from 70 patients with invasive breast cancer that discontinued their treatment due to progressive disease, as well as from five different breast cancer mouse models, and they enriched for CTCs using the Parsortix microfluidic device (Angle PLC, United Kingdom). Live CTCs were stained for cancer-associated cell surface markers EpCAM, HER2, and EGFR or imaged directly for the expression of GFP, as well as labeled for CD45 to identify WBCs. Among 70 patients, 34 (48.6%) had detectable CTCs, with a mean number of 22 CTCs per 7.5ml of blood per patient. While the majority of CTCs were single (88.0%), the inventors also detected CTC clusters (8.6%) and CTC-WBC clusters (3.4%) (**Fig. 1a**). Similarly, the inventors observed that CTC-WBC clusters were present in all tested mouse models, comprising those with immunodeficient and immunocompetent background, ranging from 0.05% to 61% of the total CTC population (**Fig. 1a, f**). Importantly, CTC abundance and ratios dramatically changed when drawing blood upstream of capillary beds as opposed to more downstream locations, indicating that the majority of clustered CTCs are already shed very early, but may be trapped in capillary beds before reaching the periphery. Capillary beds are typically found in organs (liver, lungs, brain, etc, etc,) as well as at the body extremities (e.g. finger tips). If the blood is drawn before it enters a capillary bed (e.g. if the blood is taken from the blood vessel that directly comes out from the tumor) much higher numbers of CTCs are observed. Thus, CTC-WBC clusters are rare in the peripheral circulation, yet consistently found across breast cancer patients and mouse models.

*Example 2:*

The inventors then asked what type of WBCs is found in CTC-WBC clusters. The inventors made use of a robotic micromanipulator to dissociate CTC-WBC clusters, enabling single-cell RNA sequencing analysis of cluster-associated WBCs and their comparison to reference WBCs from matched donors using reference component analysis (RCA) (Li, H. et al. Nat Genet 49, 708-718, doi:10.1038/ng.3818 (2017)). In patients, the inventors found that 75% of CTC-associated WBCs clustered to the myeloid lineage, while the remaining ones (25%) were similar to T cells. Similarly, the inventors found that 93% of CTC-associated WBCs from mouse models were also characterized by a myeloid cell-like expression profile (**Fig. 1b**). To dissect the exact proportion of CTC-associated WBCs that were neutrophils, monocytes or macrophages, the inventors labeled CTC-WBC clusters for Ly-6G, CD11b, F4/80, as well as with Wright-Giemsa staining to define nuclear morphology. The inventors found that the vast majority (85.5-91.7%) of CTC-associated WBCs were positive for Ly-6G and displayed a nuclear morphology typical of neutrophils, while a minority (8.3-14.5%) were monocytes (CD11b$^+$/F4/80$^-$/Ly-6G$^-$) and no F4/80$^+$ macrophages were found (**Fig. 1c**). Further, RNA sequencing analysis also revealed *ARG1, CXCL1, CXCL2, CXCL10, CCL2, CXCR2* and *VEGFA* expression in most CTC-associated neutrophils from both patients and mouse models (**Fig. 2d**), indicating that CTC-associated neutrophils share gene expression features of pro-tumor N2-like immune cells.

*Example 3:*

The inventors next asked whether the presence of CTC-neutrophil clusters in breast cancer patients could predict disease outcome. Strikingly, patients in whom at least one CTC-neutrophil cluster was detected in 7.5ml of peripheral blood were characterized by a significantly worse progression-free survival (PFS) (*P*=0.009) compared to patients with ≥ 5 CTCs per 7.5ml of peripheral blood (previously defined as a threshold for adverse outcome (Cristofanilli, M. et al. N Engl J Med 351, 781-791, (2004)) (**Fig. 1d**), as well as when compared to all patients with no CTC-neutrophil clusters, patients with at least one CTC per 7.5ml of blood, or patients in whom either single CTCs or CTC clusters were detected (**Fig. 3d-f**). Additionally, the inventors individually micromanipulated equal numbers of CTCs from CTC-neutrophil clusters, CTC clusters and single CTCs, spontaneously generated from tumor-bearing mice, and intravenously injected 100 CTCs per mouse in tumor-free recipient mice from each of these categories. The inventors found that mice injected with CTCs from CTC-neutrophil clusters developed overt metastasis much faster than those injected with CTCs alone, and accordingly, survived less ($P<0.0001$) (**Fig. 1e**). Thus, CTC-neutrophil clusters represent the most efficient metastasis-forming cell subpopulation among breast CTCs, and their presence in the bloodstream of patients is associated with a poor prognosis.

*Example 4:*

The inventors next sought to determine the molecular consequences of the interaction between CTCs and neutrophils by dissociating CTC-neutrophil clusters and comparing the expression profile of CTCs from CTC-neutrophil clusters to that of CTCs alone. The inventors first determined differential gene expression in the Balb/c-4T1-*GFP* model, where they could retrieve the highest number of CTCs from CTC-neutrophil clusters ($n=24$). Compared to CTCs that were not associated to neutrophils, the inventors found that CTCs from CTC-neutrophil clusters are characterized by differential expression of a set of 51 genes (q<0.05), of which 41 are upregulated and 10 are downregulated (**Tables 1,2**). Pathway analysis with the upregulated genes revealed that CTCs from CTC-neutrophil clusters display a remarkable enrichment in positive regulators of cell cycle and DNA replication programs compared to CTCs alone (q<0.05), independently of the number of reads, homogeneity or detected features in each individual sample. The same results were also obtained from CTCs of patients. Immunofluorescence staining of CTCs confirmed higher levels of Ki67 expression in CTCs from CTC-neutrophil clusters (**Fig. 2a**), in line with the RNA sequencing results. In contrast, no significant differences were observed for genes involved in epithelial-to-mesenchymal transition, cancer stem cell markers, or platelet-related genes. Further, the inventors also asked whether neutrophil proximity would confer a proliferative advantage to cancer cells at the level of the primary tumor, disseminated tumor cells (DTCs) and overt metastasis. Surprisingly, the inventors found that Ki67 expression does not increase in cancer cells that surround tumor-infiltrated neutrophils in the primary tumor or overt metastasis. Yet, a higher Ki67 expression is retained by CTCs from CTC-neutrophil clusters upon dissemination (**Fig. 2b**), i.e. when they are deprived of other stromal-derived signals that are typical of overt disease, and leading to an increased ability to successfully seed metastasis.

*Example 5:*

The inventors then asked which cytokines are expressed by CTC-associated neutrophils and paralleled by simultaneous expression of their matching cytokine receptor(s) in the corresponding cancer cells. The inventors found that four cytokines (*TNFα, OSM, IL1β,* and *IL6*) are most frequently expressed by CTC-associated neutrophils of patients or patient-derived mouse models, and matched by the expression of their receptors by the corresponding cancer cells (**Fig. 5a**). With a reverse approach, the inventors also found that CTCs from CTC-neutrophil clusters most frequently express cytokines such as *CSF1, CSF3* (a.k.a. granulocyte colony-stimulating factor, G-CSF), *TGFβ3*, and *IL15,* possibly involved in neutrophil stimulation, while corresponding neutrophils express their receptors (**Fig. 5b**). Consistently, the inventors observed that a 24h *in vitro* treatment (coherent with neutrophil lifespan) with IL6, IL1β or both was sufficient to confer proliferative advantage to 4T1 cells upon dissemination, leading to a faster metastasis development and shorter overall survival of mice (**Fig. 2c** and **Fig. 5c-e**). Conversely, CRISPR-mediated knockout of IL6 or IL1β receptors on cancer cells, namely IL6ST and IL1R1, did not alter the frequency of spontaneously-generated CTC-neutrophil clusters but it suppressed their proliferative advantage (**Fig. 5f-h**).

*Example 6:*

Given recent findings highlighting that the presence of myeloid cells in the primary tumor site leads to accumulation of mutational events, the inventors asked whether the mutational load of CTCs obtained from CTC-neutrophil clusters was different from that of CTCs alone in patients. Interestingly, the inventors found that the mutational burden was similar both between CTCs isolated from CTC-neutrophil clusters and CTC alone, as well when comparing donors with or without CTC-neutrophil clusters. Yet, the inventors observed that the overall frequency of C>T mutations was increased, while the frequency of C>G was decreased in CTCs from CTC-neutrophil clusters compared to CTCs alone, as well as in donors with CTC-neutrophil clusters, with changes in mutation frequencies being independent of the nucleotide context (**Fig. 3a**). While a general, age-related accumulation of C>T mutations has been reported, the inventors did not observe any age difference between the two groups. Next, considering only high-impact mutations, the inventors asked whether specific genes were exclusively and recurrently mutated in donors with CTC-neutrophil clusters. This scenario would be consistent with a model whereby certain genetic alterations would influence the recruitment of immune cells to the primary tumor, and increase the likelihood of generating CTC-neutrophil clusters. The inventors found that a number of genes were indeed carrying high-impact mutations exclusively in donors with CTC-neutrophil clusters (**Fig**

**3g and Table 3**). The inventors then engineered 4T1-*GFP* cells to individually express all the mutations found in two of the most frequently mutated genes, i.e. *MERTK* and *TLE1,* and injected them in the mammary gland of NSG mice. The inventors observed that the introduction of *TLE1* mutations 1787G>A or 1509G>C led to a higher infiltration of neutrophils in the primary tumor (**Fig. 3b**) and a higher proportion of spontaneously-generated CTC-neutrophil clusters (33-41-fold increase; see **Fig. 3c**), without affecting primary tumor size (**Fig. 6a-b**). These results are in line with recent observations involving *TLE1* function in regulating myeloid cells infiltration into normal and neoplastic tissues. In contrast, co-culture of cancer cells with neutrophils did not result in the acquisition of mutations within the same hotspots. Together, the inventors' data revealed that while the overall mutational load remains unchanged, donors with CTC-neutrophil clusters feature a higher proportion of C>T substitutions and the presence of high-impact recurrent mutations in genes that promote neutrophil recruitment, such as *TLE1.*

*Example 7:*

The inventors next tested whether the depletion or augmentation of the total neutrophil population would affect the ratio of spontaneously-generated CTC-neutrophil clusters. The inventors depleted neutrophils by *in vivo* treatment with neutralizing antibodies against Ly-6G ($\alpha$Ly-6G) or conversely, the inventors stably overexpressed G-CSF to stimulate the production and recruitment of neutrophils to the tumor site. As expected, while treatment with $\alpha$Ly-6G reduced neutrophil infiltration to the primary tumor site, G-CSF augmented it without altering primary tumor size. Yet, $\alpha$Ly-6G-treated mice completely lacked CTC-neutrophil clusters from the circulation and displayed a delayed CTC shedding rate compared to control mice, while overexpression of G-CSF led to an earlier CTC release and substantially increased the proportion of CTC-neutrophil clusters (>88-fold) (**Fig. 4a**). As a consequence, neutrophil depletion or augmentation resulted in a delayed or accelerated metastasis development, respectively (**Fig. 4b**), mirrored by a shorter or longer overall survival of treated mice. Instead, neutrophil depletion with $\alpha$Ly-6G was not effective when cancer cells were administered directly through intravenous injection of pretreated mice. Of note, G-CSF treatment of breast cancer patients was also more frequent in those patients that were positive for CTCs, including CTC-WBC clusters. Thus, overall neutrophil abundance impacts on the likelihood that a tumour spontaneously sheds CTC-neutrophil clusters.

*Example 8:*

Beyond changes in overall neutrophil numbers, the inventors sought to identify actionable vulnerabilities of CTC-neutrophil clusters without targeting the entire neutrophil population. To this end, the inventors found cell-cell junction pairs expressed by CTC-neutrophil clusters and possibly mediating their heterotypic cell binding. The inventors then engineered a CRISPR/Cas9-based loss-of-function minipool screen *in vivo,* whereby a pool of cells carrying individual knockouts for F11R, ICAM1, ITGB2 and VCAM1 are injected in the mammary gland of recipient mice, followed by targeted barcode sequencing of CTCs to reveal selective sgRNA dropouts, highlighting genes whose knockout would not allow CTC-neutrophil clusters formation. Importantly, the inventors observed no differences in primary tumor growth and no selective sgRNA dropouts in primary tumor cells, suggesting that knockout of F11R, ICAM1, ITGB2 or VCAM1 does not affect proliferation at the level of the primary tumor. Yet, the inventors found that 4/4 VCAM1 sgRNAs selectively dropped out in CTCs from CTC-neutrophil clusters, while they were still present in CTCs alone (**Fig. 4c**), suggesting VCAM1 requirement for CTC-neutrophil clusters formation. This experiment also shows that the other F11R, ICAM1, ITGB2 are likely to be involved in CTC-neutrophil cluster formation, but to a minor extent than VCAM1 in this particular model. The inventors further validated this finding using sgRNAs individually (**Fig. 4d**). Thus, VCAM1 functionally mediates the interaction between CTCs and neutrophils, and its inhibition does not allow the formation of CTC-neutrophil clusters.

*Discussion*

Altogether, the inventors' data provide new insights into the processes that define the interaction between cancer cells and immune cells during blood-borne dissemination and at single-cell resolution. The inventors propose a model whereby neutrophils directly interact with CTCs to support cell cycle progression in circulation and to accelerate metastasis seeding. This mechanism of metastatic spread and the possibility that CTC-neutrophil-clusters may be targeted therapeutically provides an opportunity to reduce the spread of breast cancer.

*Materials and methods*

*Patients*

After obtaining written informed consent, breast cancer patients donated 7.5 -15ml of blood in EDTA vacutainers at least once. All blood specimens were obtained at the University Hospital Basel under the study protocols EKNZ BASEC 2016-00067 and EK 321/10, approved by the Swiss authorities (EKNZ, Ethics Committee northwest/central Switzerland) and in compliance with the Declaration of Helsinki. Involved patients were characterized by invasive breast cancer (all subtypes), high tumor load and progressive disease at the time of blood sampling.

*Cell culture*

MDA-MB-231 LM2 human breast cancer cells (obtained from Dr. Joan Massagué, MSKCC, NY, USA) and 4T1 murine breast cancer cells (ATCC) were grown in DMEM medium (#11330-057, Gibco) supplemented with 10% FBS (#10500064, Gibco) and antibiotic/antimycotic (#15240062, Gibco) in a humidified incubator at 37 °C with 20% $O_2$ and 5% $CO_2$. Py2T cells were a gift from Dr. Gerhard Christofori. Human CTC-derived BR16 cells were generated as previously described (Yu et al. Science 345, 216-220, 2014.) and propagated as suspension cultures in a humidified incubator at 37 °C with 5% $O_2$ and 5% $CO_2$. All cell lines were transduced with lentiviruses carrying GFP-Luciferase (*GFP*) at a multiplicity of infection (MOI) < 5.

*Mouse experiments*

All mouse experiments were carried out according to institutional and cantonal guidelines (approved mouse protocol #2781, cantonal veterinary office of Basel-City). Nod Scid Gamma (NSG) and Balb/c mice were purchased from The Jakson Laboratory (Bar Harbor, Maine, USA) and kept in pathogen-free conditions, accordingly to institutional guidelines. Transgenic MMTV-PyMT mice were obtained from Dr. Gerhard Christofori (University of Basel). Orthotopic breast cancer lesions were generated in 8-10 weeks old NSG females upon the injection with either $1x10^6$ LM2-*GFP*, $0.5x10^6$ *4T1-GFP* or $1x10^6$ BR16-*GFP* cells into the mammary fat pad. Similarly, Balb/c mice received a syngeneic graft of $0.5x10^6$ 4T1-*GFP* cells. In all cases, breast cancer cells were inoculated in 100$\mu$l of 50% Cultrex PathClear Reduced Growth Factor Basement Membrane Extract (#3533-010-02, R&D Biosystems) in PBS. Blood draw for CTC analysis and organ dissection were performed after 3 weeks for NSG-4T1-*GFP,* 4-5 weeks for Balb/c-4T1-*GFP* and NSG-LM2-*GFP*, 5 months for NSG-BR16-*GFP* and at 13 weeks of age for MMTV-PyMT mice.

*CTC capture*

Human CTCs were captured from unprocessed peripheral blood samples with the Parsortix microfluidic device using Cell Separation Cassettes (GEN3D6.5, ANGLE), within 1 hour from blood draw. Next, in-cassette staining was performed with an antibody cocktail containing antibodies against EpCAM-AF488 (#CST5198, Cell Signaling Technology), HER2-AF488 (#324410, BioLegend), EGFR-FITC (#GTX11400, GeneTex) and CD45-BV605 (#304042, BioLegend). For mouse experiments, 500-1000$\mu$l of blood was collected through cardiac puncture and processed immediately on the Parsortix microfluidic device. For tumor-draining vessel experiments, the tumor was first exposed by opening the mouse flank. The largest tumor-associated vessel was then localized and approximately 2$\mu$l of blood was collected upon a small incision. CTCs from the MMTV-PyMT mouse model were stained with antibodies against mouse EpCAM-AF488 (#118210, BioLegend) and CD45-BV605 (#103140, BioLegend). For all other models (xenografts and syngeneic), carrying cancer cells stably expressing a GFP-Luciferase reporter, only anti-CD45 staining was performed, while CTCs were identified based on GFP expression. The number of captured CTCs, including single CTCs, CTC clusters and CTC-WBC clusters, was determined while cells were still in the cassette. CTCs were then released from the cassette in DPBS (#14190169, Gibco) onto ultra-low attachment plates (#3471-COR, Corning). Representative pictures were taken at 40x magnification with Leica DMI4000 fluorescent microscope using Leica LAS and analyzed with ImageJ.

*Assessment of the direct metastatic potential of CTCs*

8-10 weeks old NSG females were injected with 500'000 4T1-*GFP* cells. Upon tumor development, blood was collected via heart puncture and run through the Parsortix device. Single CTCs, CTC clusters and CTC-neutrophil clusters were individually micromanipulated and 100 cells per mouse from each category were injected into the tail vein of recipient NSG females. Metastasis onset and growth rate in lungs was noninvasively monitored on a weekly schedule with the IVIS system.

*White blood cell sorting*

Reference WBCs were obtained from the peripheral blood of breast cancer patients (*n*=5) and healthy individuals (*n*=3) after signing informed consent, naïve NSG and Balb/c mice (females at 8-12 weeks), Balb/c-4T1-*GFP* and NSG-CDX-BR16-*GFP* mouse models at the time of experiment termination. In brief, red blood cells, granulocytes and mononuclear cells were separated by gradient centrifugation with Lymphoprep (#1114545, STEMCELL Technologies). Desired fractions were manually isolated and washed with 1% BSA/PBS buffer. Additionally, the granulocyte fraction was purified from contaminating red blood cells by 10 minutes incubation in 0.16mol/L ammonium chloride. Unspecific antibody binding was prevented by blocking the Fc receptor for 15 minutes (human: #422301, BioLegend; mouse: #101320, BioLegend). Cells were stained with white blood cell markers: human - anti-CD14-APC (#301808, BioLegend), anti-CD66b-FITC (#305104, BioLegend), anti-CD3-BV421 (#317344, BioLegend), anti-CD19-FITC (#302206, BioLegend), anti-CD335-PE (#331908, BioLegend) anti-CD41-PE/Cy5 (#303708, BioLegend); mouse - anti-Gr-1-APC/Cy7 (#108423, BioLegend), anti-CD11b-APC (#101211, BioLegend), anti-CD3-BV421 (#100227, BioLegend), anti-CD19-FITC (#115505, BioLegend) or anti-CD19-BV605 (#115539, BioLegend; for mouse models with GFP reporter), anti-CD49b-PE (#108907, BioLegend), anti-CD41-PE/Cy5 (#133921, BioLegend). Cell populations were determined by the expression of characteristic markers: for human granulocytes ($CD66b^+CD41^-$), monocytes ($CD14^+CD3^-CD19^-CD335^-CD41^-$), T cells ($CD14^-CD3^+CD19^-CD335^-CD41^-$), B cells ($CD14^-CD3^-CD19^+CD335^-CD41^-$), NK cells ($CD14^-CD3^-CD19^-CD335^+CD41^-$); for mouse granulocytes ($Gr-1^+CD41^-$), monocytes ($CD11b^+CD3^-CD19^-CD49b^-CD41^-$), T cells ($CD11b^-CD3^+CD19^-CD49b^-CD41^-$), B cells ($CD11b^-CD3^-CD19^+CD49b^-CD41^-$), NK cells ($CD11b^-CD3^-CD19^-CD49b^+CD41^-$). One hundred cells from each population were sorted (FACSAria III, BD Biosciences) directly into microcentrifuge tubes containing 2.5$\mu$l RLT Plus lysis buffer (#1053393, Qiagen).

*Neutrophil co-culture with tumor cells*

Human neutrophils were purified from healthy donor blood upon gradient centrifugation with Lymphoprep™ (Stemcell Technologies). 8'000 neutrophils were added to 100'000 BR16 or Brx50 CTC-derived cells and co-cultured in CTC media for 72 hours. Then, gDNA was isolated from tumor cells and submitted for whole exome sequencing.

*Exome and Transcriptome Sequencing*

Individual cells from CTC-WBC clusters were mechanically separated with gentle micromanipulation (Cell-Celector, ALS). AF488/FITC-positive (or GFP-positive) and BV605-negative CTCs or AF488/FITC-negative and BV605-positive WBCs were immediately transferred into individual tubes (#321-032-501, Axygen) containing 2.5$\mu$l RLT Plus lysis buffer (#1053393, Qiagen) and 1U/$\mu$l SUPERase In RNase Inhibitor (#AM2694, Invitrogen). Samples were immediately frozen on dry ice and kept at -80°C until further processing. Following previously published protocol for parallel DNA and RNA sequencing from individual cells (Macaulay et al. Nat Protoc 11, 2081-2103, (2016).), genomes and transcriptomes of lysed cells were separated and amplified (#25-6601-97, GE Healthcare for genome and Smart-seq2 from for transcriptome). Reference white blood cells were prepared solely with Smart-seq2 protocol. Libraries were prepared with Nextera XT (Illumina), exomes were enriched using SureSelect XT Human All Exon v6 + Cosmic kit (Agilent technologies) and sequenced on HiSeq 2500 (Illumina) in 100bp paired-end mode for DNA sequencing and on NextSeq 500 (Illumina) 75bp single read mode for RNA sequencing.

*Differential white blood cell staining on CTC-WBC clusters*

Live CTCs captured within the Parsortix microfluidic cassette were stained with anti-Biotin-CD45 (#103104, BioLegend) and detected with Streptavidin-BV421 (#405226, BioLegend), anti-mouse Ly-6G-AF594 (#127636, BioLegend) and anti-CD11b-AF647 (clone M1/70, kind gift from Dr. Roxane Tussiwand, University of Basel) or with anti-F4/80-AF594 (#123140, BioLegend) and CD11b-AF647. Additionally, MMTV-PyMT-derived CTCs were marked with EpCAM-AF488 (#118210, BioLegend). Next, cells were gently released from the microfluidic system into ultra-low attachment plate and immediately imaged (Leica DMI400). The number of CTC-WBC-clusters with neutrophils ($Ly-6G^+CD11b^{med}$), monocytes ($Ly-6G^-CD11b^{med/high}$) and macrophages ($F4/80^+CD11b^+$) was assessed. Immediately after imaging, cells were centrifuged (500rpm, 3 minutes) on a glass slide and fixed in methanol for 1 minute. After brief air-drying, slides were stained using Wright-Giemsa stain kit (#9990710, ThermoFisher) to visualize nuclear morphology of captured cells, following the manufacturer's instructions.

*Immunofluorescence staining*

Formalin-fixed, paraffin-embedded (FFPE) sections were obtained from primary tumors and metastatic sites of two patients with ER/PR-positive breast cancer (Department of Pathology, University Hospital Basel) who had detectable CTC-WBC clusters. Similarly, mouse-derived primary tumors and metastases were fixed in 4% paraformaldehyde and prepared according to a standard paraffin embedding protocol. Human and mouse sections were handled according to a standard immunofluorescent paraffin-embedded tissue staining protocol. Briefly, after deparaffinization in xylene and re-hydratation, antigen retrieval was carried out in 10mmol/L sodium citrate (pH 6.0) at 95°C for 25 minutes.

For CTC and DTC staining, cell suspension was centrifuged (3min, 500 rpm) on a coated glass slide (#5991056, ThermoFisher) and air-dried. Cells were fixed in 4% paraformaldehyde for 12 min and stored in PBS until needed. For both FFPE sections and cells, after 1 hour of blocking with 10% horse serum, specimens were co-stained for pan-cytokeratin (#GTX27753, Genetex) detected with anti-mouse IgG-AF488 (#A-21202, ThermoFisher), anti-myeloperoxidase (#AF3667-SP, R&D) detected with anti-goat IgG-AF568 (A-11057, ThermoFisher), Ki67 (#ab15580, Abcam) detected with anti-rabbit IgG-AF647 (A-31573, ThermoFisher) and DAPI (#D1306, ThermoFisher).

*In vitro cytokine treatment*

100'000 *4T1-GFP* cells per well were seeded in a 6-well plate and cultured in growth medium overnight. Next morning, cells were washed 3 times with PBS and given starvation medium (0.1% FBS). After 48h, the medium was supplemented with 25ng/ml recombinant mouse IL-6 (#575702, BioLegend), IL-1$\beta$ (#575102, BioLegend), TNF$\alpha$ (#575202, BioLegend) and OSM (#762802, BioLegend), either individually or in combination. Cells were stimulated for 24h and then harvested upon trypsinization, enumerated using automatic cell counter and 300'000 cells were injected intravenously into 8-10 weeks old female mice.

*Mertk and Tle1 mutagenesis*

Lentiviral vectors with human Mertk (CCSB-Broad LentiORF, CloneId: ccsbBroad304_11503, Dharmacon) and human Tle1 (Precision LentiORF, CloneId: PLOHS_100005903, Dharmacon) served as base for introduction of specific mutations using QuikChange II XL site-directed mutagenesis kit (#200522, Agilent Technologies). Lentiviral particles were then prepared with Dharmacon Transduction Starter Kit and upon transduction, 4T1-GFP cells were selected with 6 $\mu$g/ml Blasticidin S for 6 days.

*Myleoid cells depletion*

For neutrophil depletion studies in primary tumor models, mice were injected intraperitoneally with 400$\mu$g of anti-Ly-6G IgG2a (#127650, BioLegend) or control IgG2a (#400566, BioLegend) when tumors were palpable (day two after injection of 4T1-*GFP* cells, day six after injection of LM2-*GFP* cells and day 30 after injection of BR16-*GFP* cells). Efficiency of immune cell depletion was monitored after 48 hours with Advia120 Hematology Analyzer (Siemens) using Multispecies version 5.9.0-MS software (Bayer). Additionally, NSG-4T1-*GFP* mice received a second dosage of anti-Ly-6G or control IgG2a antibodies (100 $\mu$g) on day 19, NSG-LM2-*GFP* mice received a second dosage on day 25 and NSG-BR16-*GFP* mice received a second dosage on day 45. Tumor size was determined with caliper measurements every seven days and tumor volume was calculated using modified ellipsoid formula: V=1/2(Length $\times$ Width$^2$). At termination, lung metastases were measured with IVIS Lumina II (Perkin Elmer) and metastatic index was determined by normalizing the photon/second count of the metastasis with that of the primary tumor. For neutrophil pre-depletion experiments, a single dose of 400$\mu$g of anti-Ly-6G IgG2a was injected intraperitoneally 24h before tumor cells i.v. inoculation. Mice were sacrificed in accordance to our approved protocol and the survival data was inferred accordingly.

*G-CSF overexpression*

Human G-CSF was transduced into *4T1-GFP,* LM2-*GFP* and BR16-*GFP* cells using the Precision LentiORF (GE Healthcare) system. Construct-positive cells were selected with 9$\mu$g/ml Blasticidin S for 4 days (4T1) or 7 days (LM2, BR16). Overexpression of G-CSF was confirmed by qPCR using human-specific primers for LM2 and BR16 cells (Forward: 5'GAGTTGGGTCCCACCTTG3' (SEQ ID NO: 001), Reverse: 5TGGAAAGCAGAGGCGAAG3' (SEQ ID NO: 002)) or primers recognizing both mouse and human transcripts for 4T1 cells (Forward: 5TGTGCCACCTACAAGCTGTG3' (SEQ ID NO: 003), Reverse: 5'CCATCTGCTGCCAGATGGTGGT3' (SEQ ID NO: 004)).

*Generation of 4T1-Cas9-GFP cells*

4T1 cells were transduced with the lentiviruses carrying pLentiCas9-*EGFP* (#63592, Addgene) at a MOI of 1. *GFP*-positive cells were sorted as single cells into 96-well plates and cultured as clonal cell lines. Lines with 100% *GFP*-positivity were kept and Cas9 expression was confirmed by western blotting (#844301, Biolegend).

*sgRNA minipool design, transduction and in vivo transplantation*

All sgRNAs for were designed using the GPP Web Portal (https://portals.broadinstitute.org/gpp/public/analysis-tools/sgrna-design) and sgRNA oligos were synthesized by Microsynth. Each sgRNA was individually cloned into the pLentiGuide-Puro vector (#52963, Addgene). 4T1-Cas9-*GFP* cells were then transduced separately with each individual sgRNA vector at MOI=0.4. Upon seven days of puromycin selection, 4T1-Cas9-*GFP* cells carrying individual sgRNAs were mixed in equal cell numbers, taken for genomic DNA extraction and, at the same time, subcutaneously injected (≥ 1000 cells per sgRNA; 500'000 total cells) into the mammary fad pad of NSG mice.

*sgRNA sequencing*

gDNA was extracted from cells at different stages (prior to injection, upon primary tumor growth and from spontaneously formed CTCs) by using salt precipitation. The library preparation was carried out using two-step PCR, where the first PCR amplifies a broad region including the sgRNA sequence cassette and the second PCR adds Illumina sequencing adapters to the products from the first PCR, as described previously (Chen et al. Cell 160, 1246-1260, (2015).). Samples were then sequenced on NextSeq 500 SR75 sequencers.

*Single-cell RNA-seq data processing*

After sequencing, initial quality assessment for RNA-seq data was performed using FastQC (https://www.bioinformatics.babraham.ac.uk/projects/fastqc), FastQ Screen (https://www.bioinformatics.babraham.ac.uk/projects/fastq_screen), and visualized with MultiQC (v0.8). Adaptor sequences, first 9 base pairs and low quality ends were removed with Trim Galore (v0.4.2, http://www.bioinformatics.babraham.ac.uk/projects/trim_galore/; parameters : --phred33length 36 --ctip_R1 9). Trimmed reads were aligned to a combined human (GRCh38) and mouse (GRCm38) genome reference using STAR (v 2.5.2a; parameters: --runMode alignReads-genomeLoad LoadAndExit). Quality control of resulting BAM files was performed with RSeQC (v2.6.4). The gene-level expression counts were computed with featureCounts (v1.5.1) using the gene annotations obtained from RefSeq (release 70). Samples with less than 800 features detected (threshold ≥ 1 mapped read) or showing more than 5% of contamination from the other species were removed from further analysis. To normalize gene counts for cell-specific biases, the inventors used size factors computed utilizing the deconvolution implemented in the scran package (v1.6.5) available on R/Bioconductor. After normalization, the effect of technical factors (library size and number of detected features) on variance was evaluated using t-distributed stochastic neighbor embedding (t-SNE) adjusted by patient or mouse model. CTCs showing a substantial contribution of stromal genes and the absence of cancer-associated genes, and CTC-associated WBCs showing no expression of CD45 were removed from the analysis. scRNA-seq data processing, quality control, and visualization was performed with the help the R/Bioconductor package scater (v 1.6.0).

*Reference Component Analysis (RCA)*

RCA was utilized to identify single cell types using reference transcriptomes. For human samples, the reference bulk transcriptomes were obtained from the Human U133A/GNF1H Gene Atlas and the Primary Cell Atlas (http://biogps.org/), averaging expression levels when multiple replicates were present. Mouse transcriptomes were obtained from the Mouse GNF1M and MOE430 Gene Atlas (http://biogps.org/). The initial gene selection for the reference transcriptome panel was performed, as previously described (Li et al. Nat Genet 49, 708-718, (2017).). An additional filtering of genes was achieved by removing genes specific to CTCs from the human panel and by selecting highly variable genes (HVGs) from the mouse panel. A gene was defined as CTC-specific if its normalized expression (log-counts) relative to the median across the reference WBCs set exceeded 5 in at least 5% of CTC samples. In mouse, only genes that showed high variability in their expression across reference WBCs were included. In order to select HVGs in mouse, gene-specific variance of expression across reference WBCs was estimated using trendVar and decomposed into biological and technical components using decomposeVar from scran package. Highly variable genes were selected on the basis of their biological component (biological variance ≥ 5) and adjusted *P*-value (threshold ≤ 0.05). A total of 5,279 genes were selected for the human reference panel and 655 for the mouse panel. Projection of each sample onto the reference transcriptome was performed as previously described (Li et al. Nat Genet 49, 708-718, (2017).), calculating the pearson correlation between the $\log_{10}$ (FPKM) values of the scRNA-seq samples and the $\log_{10}$ expression values of the global panel using the functions provided by the RCA R package (v1.0; https://github.com/GIS-SP-Group/RCA). For visualization, reference cell types with a low correlation with query samples and non-immune related features were removed. Hierarchical clustering was performed to cluster samples based on their projection values.

*Differential expression and gene set enrichment analyses*

The inventors determined differentially expressed genes by the edgeR likelihood ratio test method (v3.20.1) using the normalized counts with the deconvolution approach and the robust dispersion of estimates options. Gene set over-representation analysis of KEGG pathways in the differentially expressed genes (adjusted p-value threshold $\leq 0.25$) was performed with the kegga method implemented in the edgeR R/Bioconductor package (v3.20.1). Enrichment of the KEGG pathways 'Cell cycle' (hsa04110) and 'DNA replication' (hsa03030) in patient samples was tested with the self-contained rotation gene set test (roast) from the limma R/Bioconductor package (3.34.2) using the msq option as a gene set summary statistic and 5000 rotations to compute p-values.

*Cytokine and cytokine ligand analysis*

A comprehensive collection of cytokines and their receptors was obtained from KEGG pathway Cytokine-cytokine receptor interaction' (accession codes hsa04060 and mmu04060 for human and mouse, respectively). Next, human one-to-one orthologous genes for the mouse gene set were obtained from Ensembl (v91) using the biomaRt (v2.34) R/Bioconductor package in order to combine human and mouse datasets. A cytokine-receptor pair was considered to be expressed in a CTC-neutrophil cluster if the cytokine gene in the neutrophil sample and its corresponding receptor in the CTC were expressed at log2 normalized counts per million mapped reads (CPM) $\geq 5$. For CTC-neutrophil clusters containing more than one detached CTC, all possible CTC-neutrophil pairs were considered.

*Single-cell DNA-seq data processing*

Paired-end reads were aligned to the GRCh38 human or GRCm38 mouse reference genomes using BWA-mem algorithm (v0.7.13; parameters : -M) (https://arxiv.org/abs/1303.3997) and sorted using SAMtools (v1.3.1). Reads were then deduplicated using Picard MarkDuplicates (v2.9.0; http://picard.sourceforge.net/) on a per-sample basis and local realignment was performed using the Genome Analysis Toolkit (GATK) IndelRealigner (v3.7.0) at the sample and donor level to improve alignment accuracy around indels. Quality control and coverage and exome enrichment statistics were generated using FastQC, CollectHsMetrics from Picard suite, and QualiMap (v 2.2.1) and visualized using MultiQC (v0.8).

*Somatic mutation calling and mutation spectrum*

Mpileup files were generated with SAMtools (v1.3.1; parameters : -B -q 40) and variants were called using Monovar (v2016-05-14) on all samples from the same donor simultaneously. Resulting variants were annotated using SnpEff on ENSEMBL v86 (www.ensembl.org), dbSNP (build 150), 1000 genomes project (phase 1), and coding mutations from cosmic (v81) using SnpSift (v4.3p). Somatic mutation rates were calculated as the ratio of the number of somatic variants and the number of nucleotides covered in the exome at $\geq 2x$. Putative damaging somatic mutations were identified exclusively in donors with matched WBC sequenced using an empirical filtering strategy removing (1) variants present in public databases (dbSNP, 1000 genomes project) at a frequency $\geq 1\%$ or found in 2 or more founders, (2) variants present in at lest one reference WBC sample from the same donor, (3) variant loci not covered in reference WBC samples (threshold $\geq 3$ reads), and (4) likely damaging events (truncating, frameshift or splice site variant). VCF processing, downstream filtering, and analysis was performed using the VariantAnnotation and vcfR R/Bioconductor packages. Trinucleotide context of the somatic mutation spectrum was generated and visualized with the SomaticSignatures package (v2.14.0).

*Survival analyses*

Survival analyses were performed using the survival R package (v 2.41-3). Kaplan-Meier curves were generated and Log-Rank test was used to estimate the significance of the difference in survival between groups. For patients, progression-free survival was defined as the period between primary tumor diagnosis and first relapse. For NSG-4T1-*GFP* mouse model analysis, death was selected as the endpoint for the analysis and defined as the moment a given animal had to be euthanized according to our mouse protocol guidelines.

*Data availability*

Data analysis, statistical testing and visualization were conducted in R (version 3.4.0; R Foundation for Statistical Computing, Vienna, Austria). RNA and exome sequencing data have been deposited to Gene Expression Omnibus (GEO, NCBI; accession number GSE109761) and to the European Nucleotide Archive (ENA, EMBL-EBI; accession number PRJEB24623), respectively. Original R scripts to reproduce data analysis have been deposited to GitHub (CA, USA; accession URL https://github.com/CMETlab/CTC-WBC).

**Table 1. Genes upregulated in CTCs from CTC-neutrophil-clusters obtained from the Balb/c-4T1 mouse model.**
Table listing the genes upregulated in CTCs from CTCneutrophil-clusters compared to CTCs alone. Gene ID, fold change, *P* value and *Q* value are shown.

| Gene ID | Fold change | *P* value | *Q* value |
|---|---|---|---|
| Coll7a1 | 7.729711543 | 4.38E-05 | 0.022963533 |
| Nxt2 | 7.271228295 | 1.69E-05 | 0.014192394 |
| Mecom | 7.397491609 | 3.77E-05 | 0.022024227 |
| Slfn3 | 7.077366966 | 1.04E-04 | 0.03905821 |
| Adck4 | 7.427678279 | 1.30E-04 | 0.042211886 |
| G630025P09Rik | 7.287674752 | 3.69E-05 | 0.022024227 |
| Tmem102 | 7.224902975 | 3.95E-05 | 0.022024227 |
| 6430548M08Rik | 4.592459238 | 5.28E-05 | 0.02659683 |
| Osgin2 | 9.337328143 | 3.58E-09 | 0.000045053 |
| Map4k2 | 4.052985433 | 3.73E-07 | 0.001174217 |
| Cerk | 6.998938853 | 7.98E-07 | 0.002008694 |
| Bcdin3d | 7.464061077 | 9.10E-06 | 0.009548896 |
| Mcm4 | 1.966913377 | 3.12E-05 | 0.022024227 |
| Mcm6 | 2.563422692 | 1.15E-04 | 0.041240492 |
| Gm19557 | 3.687694485 | 8.98E-05 | 0.038458772 |
| Paqr4 | 2.968014763 | 9.96E-07 | 0.002089226 |
| Cczl | 1.336601221 | 1.47E-04 | 0.042232275 |
| Pts | 3.629387912 | 5.73E-05 | 0.027734751 |
| LOC100861795 | 1.925876446 | 1.34E-04 | 0.042211886 |
| Mad211 | 2.047787406 | 1.05E-04 | 0.03905821 |
| Dhx33 | 2.884231863 | 1.98E-04 | 0.049080981 |
| 2410016O06Rik | 3.79735797 | 1.43E-04 | 0.042232275 |
| Tmppe | 2.590383285 | 1.88E-04 | 0.049080981 |
| Kcnab2 | 1.503120971 | 1.60E-04 | 0.04369843 |
| Dpm2 | 1.653716335 | 1.99E-04 | 0.049080981 |
| Faml88b | 7.705872446 | 8.17E-06 | 0.009349097 |
| Wdr76 | 5.585718202 | 1.46E-04 | 0.042232275 |
| Ipp | 7.760431523 | 1.23E-05 | 0.011095049 |
| Kctd18 | 4.237152031 | 1.22E-04 | 0.042211886 |
| LOC100862377 | 8.145983123 | 1.52E-08 | 0.000095428 |
| Cln8 | 6.156148498 | 5.46E-08 | 0.000229294 |
| Zfp768 | 7.851069906 | 1.85E-06 | 0.002905122 |
| Ranbp9 | 2.990168313 | 1.53E-04 | 0.042834776 |
| Zfp606 | 7.328783228 | 4.02E-05 | 0.022024227 |
| Orc6 | 2.099485226 | 2.96E-05 | 0.022024227 |
| Dhx58 | 7.966691124 | 1.33E-04 | 0.042211886 |
| Orcl | 4.669427894 | 3.22E-06 | 0.004508625 |
| Apitdl | 2.609215464 | 1.75E-04 | 0.046756691 |
| Smpd2 | 2.810383775 | 3.62E-05 | 0.022024227 |
| Gins2 | 2.955913697 | 1.17E-05 | 0.011095049 |
| Hells | 2.268046896 | 7.26E-05 | 0.03383987 |

**Table 2. Genes downregulated in CTCs from CTC-neutrophil-clusters obtained from the Balb/c-4T1 mouse model.** Table listing the genes downregulated in CTCs from CTC neutrophil-clusters compared to CTCs alone. Gene ID, fold change, *P* value and *Q* value are shown.

| Gene ID | Fold change | *P* value | *Q* value |
|---|---|---|---|
| 9430083A17Rik | -7.011577924 | 3.55E-05 | 0.022024227 |
| Cnn2 | -1.039965548 | 1.94E-04 | 0.049080981 |
| LimaI | -1.928691973 | 9.52E-05 | 0.038647688 |
| FblimI | -1.66298953 | 6.49E-06 | 0.00816999 |
| PdlimS | -1.510923036 | 1.48E-04 | 0.042232275 |
| PlekhnI | -1.993109176 | 7.76E-05 | 0.03487739 |
| Slc40a1 | -7.047808867 | 1.68E-06 | 0.002905122 |
| H2-T23 | -1.411196227 | 1.00E-04 | 0.03905821 |
| 6330403M23Rik | -2.306668723 | 9.16E-05 | 0.038458772 |
| Lamc3 | -6.11620345 | 1.24E-04 | 0.042211886 |

**Table 3**. Genes frequently mutated in CTC-neutrophil donors. Table listing genes mutated in 50% of donors with CTC-neutrophil-clusters. High-impact mutations, as well as their predicted effects, are shown across different donors.

| Gene ID | Mutation | Donor ID | Effect |
|---|---|---|---|
| MERTK | c.2466C>T | Br23 | structural interaction variant |
| MERTK | c.2173G>A | Br57 | structural interaction variant |
| MERTK | c.1816C>T | Br61 | structural interaction variant |
| MERTK | c.1994C>T | Br61 | structural interaction variant |
| MERTK | c.1995A>G | Br61 | structural interaction variant |
| MERTK | c.2007A>G | Br61 | structural interaction variant |
| MERTK | c.2031G>A | Br61 | structural interaction variant |
| TLE1 | c.1908G>A | Br57 | structural interaction |
| TLE1 | c.1787G>A | Br30 | structural interaction |
| TLE1 | c.1551T>C | Br61 | structural interaction |
| TLE1 | c.1509G>C | Br61 | structural interaction |
| TLR9 | c.1219C>T | Br23 | stop_gained |
| TLR9 | c.1619C>A | Br30 | stop_gained |
| TLR9 | c.1345C>T | Br61 | stop_gained |
| TLR9 | c.893G>A | Br61 | stop_gained |
| TRBV6-1 | c.340G>T | Br23 | stop_gained |
| TRBV6-1 | c.340G>T | Br30 | stop_gained |
| TRBV6-1 | c.340G>T | Br57 | stop_gained |
| TTN | c.102513C>G | Br23 | stop_gained |
| TTN | c.97590C>G | Br23 | stop_gained |
| TTN | c.88895-1G>A | Br57 | splice_acceptor_variant&intron_variant |
| TTN | c.83972-1G>A | Br57 | splice_acceptor_variant&intron_variant |
| TTN | c.21067C>T | Br57 | stop_gained |
| TTN | c.20116C>T | Br57 | stop_gained |
| TTN | c.97019G>A | Br61 | stop_gained |
| TTN | c.92096G>A | Br61 | stop_gained |
| TTN | c.70737G>A | Br61 | stop_gained |
| TTN | c.65814G>A | Br61 | stop_gained |
| TTN | c.52315A>T | Br61 | stop_gained |
| TTN | c.47392A>T | Br61 | stop_gained |
| TTN | c.31312C>T | Br61 | stop_gained |

(continued)

| Gene ID | Mutation | Donor ID | Effect |
|---|---|---|---|
| TTN | c.30361C>T | Br61 | stop_gained |
| ABCA13 | c.12412C>T | Br23 | stop_gained |
| ABCA13 | c.13285C>T | Br61 | stop_gained |
| ABCC1 | c.2530G>A | Br23 | structural interaction variant |
| ABCC1 | c.2500C>T | Br57 | stop_gained |
| ADGB | c.708G>A | Br23 | stop_gained |
| ADGB | c.1707+1G>A | Br61 | splice_donor_variant&intron_variant |
| AKR1D1 | c.513T>C | Br30 | structural interaction |
| AKR1D1 | c.518G>A | Br61 | structural interaction |
| APOB | c.13188G>A | Br23 | stop_gained |
| APOB | c.2242C>T | Br23 | stop_gained&splice_region_variant |
| APOB | c.693+1 G>C | Br57 | splice_donor_variant&intron_variant |
| CENPH | c.595C>T | Br30 | stop_gained |
| CENPH | c.652-1G>A | Br57 | splice_acceptor_variant&intron_variant |
| CERK | c.430G>T | Br57 | stop_gained |
| CERK | c.433 C>T | Br61 | stop_gained |
| CLCN1 | c.979+1G>T | Br57 | splice_donor_variant&intron_variant |
| CLCN1 | c.44G>A | Br61 | stop_gained |
| CSF1R | c.1294C>T | Br23 | structural interaction variant |
| CSF1R | c.1665C>T | Br61 | structural interaction variant |
| DDB 1 | c.256G>A | Br23 | structural interaction variant |
| DDB1 | c.2103T>A | Br61 | structural interaction variant |
| DDB1 | c.980G>A | Br61 | protein_protein_contact |
| DDX47 | c.441A>T | Br57 | structural interaction variant |
| DDX47 | c.514G>A | Br61 | structural interaction variant |
| DECR1 | c.271C>T | Br57 | structural interaction variant |
| DECR1 | c.418-1G>A | Br61 | splice_acceptor_variant&intron_variant |
| DLGAP1 | c.2725-1G>A | Br57 | splice_acceptor_variant&intron_variant |
| DLGAP1 | c.955C>T | Br61 | stop_gained&splice_region_variant |
| DNAH8 | c.8137-2A>T | Br30 | splice_acceptor_variant&intron_variant |
| DNAH8 | c.1294-2A>G | Br61 | splice_acceptor_variant&intron_variant |
| DSP | c.754G>A | Br30 | structural interaction |
| DSP | c.1990C>T | Br61 | stop_gained |
| DUSP5 | c.988C>T | Br57 | stop_gained |
| DUSP5 | c.633C>T | Br61 | structural interaction |
| DUSP5 | c.792G>A | Br61 | structural interaction |
| DUSP5 | c.799A>G | Br61 | structural interaction |
| DUSP5 | c. 801 C>T | Br61 | structural interaction |
| DUSP5 | c.807T>G | Br61 | structural interaction |
| DUSP5 | c.903G>T | Br61 | structural interaction |
| EIF5 | c.932A>G | Br57 | structural interaction |
| EIF5 | c.808C>T | Br61 | structural interaction |

(continued)

| Gene ID | Mutation | Donor ID | Effect |
|---|---|---|---|
| EPG5 | c.2716C>T | Br30 | stop_gained&splice_region_variant |
| EPG5 | c.1174C>T | Br61 | stop_gained |
| GTF3C1 | c.4540C>T | Br30 | stop_gained |
| GTF3C1 | c.2351-1G>A | Br61 | splice_acceptor_variant&intron_variant |
| GUCY1A3 | c.1402C>T | Br23 | stop_gained |
| GUCY1A3 | c.973G>T | Br61 | stop_gained |
| IDE | c.949T>A | Br57 | structural interaction |
| IDE | c.1140C>T | Br61 | structural interaction variant |
| IFIT1B | c.3G>A | Br57 | start_lost&splice_region_variant |
| IFIT1B | c.414T>A | Br61 | stop_gained |
| IGSF6 | c.1A>G | Br23 | start lost |
| IGSF6 | c.201G>A | Br61 | stop_gained |
| IQGAP3 | c.2242C>T | Br23 | stop_gained |
| IQGAP3 | c.4111C>T | Br61 | stop_gained |
| KDM4A | c.318C>A | Br30 | stop_gained |
| KDM4A | c.98A>G | Br61 | structural interaction |
| KIF13A | c.3609-1G>A | Br30 | splice_acceptor_variant&intron_variant |
| KIF13A | c.186G>A | Br61 | stop_gained |
| KIF6 | c.252-1G>C | Br23 | splice_acceptor_variant&intron_variant |
| KIF6 | c.766C>T | Br61 | stop_gained |
| LRP 1 | c.8308+1G>A | Br44 | splice_donor_variant&intron_variant |
| LRP1 | c.2623G>T | Br57 | stop_gained |
| MCF2L2 | c.1959G>A | Br30 | stop_gained |
| MCF2L2 | c.878+1G>A | Br61 | splice_donor_variant&intron_variant |
| MDC1 | c.5681A>G | Br57 | structural interaction variant |
| MDC1 | c.2422C>T | Br61 | stop_gained |
| MGA | c.950C>G | Br23 | stop_gained |
| MGA | c.4097C>G | Br61 | stop_gained |
| MUC16 | c.21001C>T | Br57 | stop_gained |
| MUC16 | c.42031C>T | Br61 | stop_gained |
| NOS2 | c.1373C>T | Br44 | structural interaction variant |
| NOS2 | c.938G>A | Br61 | structural interaction |
| NOTCH2 | c.6967C>T | Br57 | stop_gained |
| NOTCH2 | c.5293C>T | Br57 | stop_gained |
| NOTCH2 | c.4688G>A | Br57 | structural interaction variant |
| NOTCH2 | c.966T>A | Br61 | stop_gained |
| NRXN1 | c.3979A>T | Br57 | stop_gained |
| NRXN1 | c.2467+1G>A | Br61 | splice_donor_variant&intron_variant |
| NUP133 | c.2295G>A | Br57 | stop_gained |
| NUP133 | c.2632C>T | Br61 | stop_gained |
| NUP 133 | c.1312C>A | Br61 | structural interaction |
| NUP214 | c.873G>A | Br23 | structural interaction variant |
| NUP214 | c.1026A>G | Br61 | structural interaction |
| NUP214 | c.1051T>C | Br61 | structural interaction |

(continued)

| Gene ID | Mutation | Donor ID | Effect |
|---|---|---|---|
| NUP214 | c.1092A>T | Br61 | structural interaction |
| NUP214 | c.1097T>C | Br61 | structural interaction |
| NUP214 | c.1099G>A | Br61 | structural interaction |
| NUP214 | c.1104C>T | Br61 | structural interaction |
| NUP214 | c.1114C>G | Br61 | structural interaction variant |
| OR10K1 | c.7C>T | Br57 | stop_gained |
| OR10K1 | c.904C>T | Br61 | stop_gained |
| PGM1 | c.1247G>A | Br23 | stop_gained |
| PGM1 | c.919G>T | Br57 | stop_gained |
| PIR | c.570G>A | Br57 | stop_gained |
| PIR | c.334C>T | Br57 | structural interaction |
| PIR | c.838A>G | Br61 | structural interaction |
| PIR | c.711C>T | Br61 | structural interaction |
| PPIH | c.275G>A | Br30 | structural interaction |
| PPIH | c.252T>C | Br61 | structural interaction variant |
| PRRC2C | c.6651+2T>C | Br57 | splice_donor_variant&intron_variant |
| PRRC2C | c.7318C>T | Br61 | stop_gained |
| PTPRB | c.4544G>A | Br23 | stop_gained |
| PTPRB | c.5881C>T | Br61 | stop_gained |
| RAB9B | c.375G>A | Br30 | structural interaction |
| RAB9B | c.451T>C | Br61 | structural interaction |
| RAB9B | c.360A>G | Br61 | structural interaction |
| RAB9B | c.312A>G | Br61 | structural interaction |
| RAB9B | c.306G>A | Br61 | structural interaction variant |
| RAB9B | c.291T>C | Br61 | structural interaction variant |
| RAB9B | c.255G>A | Br61 | structural interaction variant |
| RAB9B | c.253T>C | Br61 | structural interaction variant |
| RAB9B | c.252C>G | Br61 | structural interaction variant |
| RAB9B | c.192A>G | Br61 | structural interaction variant |
| RAB9B | c.168A>G | Br61 | structural interaction variant |
| RAB9B | c.33 T>C | Br61 | structural interaction |
| RAB9B | c.18G>T | Br61 | structural interaction |
| RELN | c.9370-2A>G | Br57 | splice_acceptor_variant&intron_variant |
| RELN | c.7606G>T | Br57 | stop_gained |
| RELN | c.4696C>T | Br61 | stop_gained |
| RNASEL | c.1322T>C | Br30 | structural interaction |
| RNASEL | c.1527G>A | Br61 | structural interaction |
| SEC24D | c.2790G>A | Br30 | stop_gained |
| SEC24D | c.1708-2A>T | Br61 | splice_acceptor_variant&intron_variant |
| SGSM1 | c.1786C>T | Br23 | stop_gained |
| SGSM1 | c.2227G>T | Br30 | stop_gained |
| SGSM1 | c.3385C>T | Br30 | stop_gained |
| SPTAN1 | c.742C>T | Br23 | stop_gained |
| SPTAN1 | c.4342C>T | Br61 | stop_gained&splice_region_variant |
| SPTB | c.4033C>T | Br23 | stop_gained |
| SPTB | c.1972C>T | Br57 | stop_gained |
| TAS2R43 | c.900G>A | Br30 | stop_gained |

(continued)

| Gene ID | Mutation | Donor ID | Effect |
|---|---|---|---|
| TRIM42 | c.43 C>T | Br57 | stop_gained |
| TRIM42 | c.1189C>T | Br61 | stop_gained |
| UNC80 | c.7388+2T>C | Br44 | splice donor variant&intron variant |
| UNC80 | c.6268-1G>T | Br61 | splice_acceptor_variant&intron_variant |
| UROD | c.504T>C | Br23 | structural interaction variant |
| UROD | c.324C>T | Br61 | structural interaction |
| USP17L17 | c.2T>G | Br30 | start lost |
| USP17L17 | c.21C>G | Br61 | stop_gained |
| USP34 | c.7735C>T | Br30 | stop_gained |
| USP34 | c.1651C>T | Br61 | stop_gained |
| USP34 | c.1441C>T | Br61 | stop_gained |
| VPS33A | c.1033C>T | Br30 | stop_gained |
| VPS33A | c.685T>C | Br61 | structural interaction |
| VPS33A | c.678T>C | Br61 | structural interaction |
| VPS33A | c.669T>G | Br61 | structural interaction |
| VPS33A | c.549A>G | Br61 | structural interaction |
| WDR89 | c.298C>T | Br23 | stop_gained |
| WDR89 | c.190C>T | Br23 | stop_gained |
| WDR89 | c.298C>T | Br57 | stop_gained |
| XRCC5 | c.888C>T | Br23 | protein_protein_contact |
| XRCC5 | c.1308A>C | Br61 | protein_protein_contact |
| XRCC5 | c.1311C>G | Br61 | protein_protein_contact |
| XRCC5 | c.1311C>G | Br61 | protein_protein_contact |
| ZNF202 | c.604C>T | Br57 | stop_gained |
| ZNF202 | c.1492A>T | Br61 | stop_gained |
| ZNF274 | c.1426C>T | Br23 | stop_gained |
| ZNF274 | c.1863C>A | Br61 | stop_gained |
| ZNF506 | c.1093G>T | Br30 | stop_gained |
| ZNF506 | c.641C>G | Br61 | stop_gained |
| ZNF585B | c.1681C>T | Br57 | stop_gained |
| ZNF585B | c.1A>G | Br61 | start lost |

## Claims

1. A non-agonist ligand specifically binding to one of

    - VCAM1 (CD106),
    - CD49d,
    - CD29,
    - IL6 receptor (CD126),
    - IL6,
    - IL1$\beta$ receptor (IL1R1),
    - IL1$\beta$,
    - F11R,
    - ICAM1 or
    - ITGB2

for use in treatment or prevention of metastatic cancer.

2. The ligand for use in treatment or prevention of metastatic cancer according to claim 1,

wherein the metastatic cancer is metastatic breast cancer.

3. The ligand for use in treatment or prevention of metastatic cancer according to any one of claims 1 or 2, wherein the ligand is an antibody, an antibody fragment, an aptamer or an antibody-like molecule.

4. The ligand for use in treatment or prevention of metastatic cancer according to any one of claims 1 to 3, wherein the ligand is a human antibody or a humanized antibody.

5. The ligand for use in treatment or prevention of metastatic cancer according to claim 4,
   wherein the ligand is selected from canakinumab, tocilizumab, and siltuximab

6. The ligand for use in treatment or prevention of metastatic cancer according to any one of the previous claims, wherein a metastatic cancer cells' capability of extravasation, infiltration into stroma of organs, survival or growth at distant sites is diminished by the ligand.

7. A nucleic acid molecule encoding the ligand according to any one of the preceding claims for use in treatment or prevention of metastatic cancer.

8. A nucleic acid molecule comprising, or consisting of, an inhibitor nucleic acid sequence capable of downregulating or inhibiting expression of a target nucleic acid sequence encoding a protein selected from:

   - VCAM1 (CD106),
   - CD49d,
   - CD29,
   - IL6 receptor (CD126),
   - IL6,
   - IL1$\beta$ receptor (IL1R1),
   - IL1$\beta$
   - F11R,
   - ICAM1 and
   - ITGB2,

   for use in treatment or prevention of metastatic cancer.

9. The nucleic acid molecule for use in treatment or prevention of metastatic cancer of claim 8, wherein said inhibitor nucleic acid sequence is able to specifically hybridize with a sequence or subsequence of

   - an exon comprised in said target nucleic acid sequence,
   - an intron comprised in said target nucleic acid sequence,
   - a promoter region modulating expression of said target nucleic acid sequence, and/or
   - an auxiliary sequence regulating expression of said target nucleic acid sequence.

10. The nucleic acid molecule for use in treatment or prevention of metastatic cancer of claim 8 or 9, wherein said inhibitor nucleic acid sequence is an antisense oligonucleotide, an siRNA, an shRNA, an sgRNA or an miRNA.

11. The nucleic acid molecule for use in treatment or prevention of metastatic cancer according to any one of claims 8 to 10, wherein the inhibitor nucleic acid sequence comprises or consists of nucleoside analogues.

12. A method for assigning a likelihood of developing metastasis to a patient, wherein a high likelihood is assigned if

   - at least one detectable CTC-neutrophil cluster is detected in a sample obtained from the bloodstream of said patient, particularly in a sample drawn from upstream of a capillary bed; and/or
   - a tumor of said patient has a mutation in gene *TLE1* selected from 1787G>A or 1509G>C; and/or
   - a tumor of said patient has a mutation in genes belonging to the frequently mutated gene list of table 3 in patients with CTC-neutrophil clusters and /or
   - more than 40%, particularly 50%, more particularly 60% of mutations inside a tumor of said patient are C>T substitutions.

13. The ligand for use in treatment or prevention of metastatic cancer in a patient according to any one of claims 1 to 6, or the nucleic acid molecule according to any one of claims 7 to 11, wherein a high likelihood of developing metastasis is assigned to said patient according to the method of claim 12.

Fig. 1

a)

| | | |
|---|---|---|
| Single CTCs | CTC-clusters | CTC-WBC clusters |

b)

Fig. 1 (continued)

c)

d)

Fig. 1 (continued)

e)

f)

Fig. 2

a)

b)

c)

Fig. 2 (continued)

d)

CTC-associated neutrophils
from mouse models

*Ptprc*
*Cxcl10*
*Cxcl1*
*Cxcl2*
*Arg1*
*Ccl2*
*Cxcr2*
*Vegfa*

CTC-associated neutrophils
from patients

*PTPRC*
*ARG1*
*VEGFA*
*CXCL10*
*CCL2*
*CXCR2*
*CXCL2*
*CXCL1*

(Projection score)

20
10
0
-10
-20

Fig. 3

a)

b)

Fig. 3 (continued)

c)

d)

Fig. 3 (continued)

e)

f)

Fig. 3 (continued)

g)

Fig. 4

Fig. 4 (continued)

Fig. 4 (continued)

Fig. 4 (continued)

c)

d)

Fig. 4 (continued)

e

*Vcam1*

Fig. 5

Fig. 5 (continued)

Fig. 5 (continued)

Fig. 5 (continued)

**g**

Ctrl sgRNA

0.1%
20.3%
79.6%

*Il1r1* KO

0.1% — sgRNA 1 — 17.6% — 82.3%
0.1% — sgRNA 2 — 15.9% — 84.0%
0.2% — sgRNA 3 — 16.8% — 83.0%

*Il6st* KO

0.1% — sgRNA 1 — 13.4% — 86.5%
0.2% — sgRNA 2 — 17.0% — 82.8%
0.2% — sgRNA 3 — 14.9% — 84.9%

☐ Single CTCs   ☐ CTC clusters
☐ CTC-neutrophil clusters

*Il1r1* KO

ns   ns   ns

Fold change

Ctrl sgRNA / *Il1r1* KO

*Il6st* KO

ns   ns   ns

Fold change

Ctrl sgRNA / *Il6st* KO

**h**

% of Ki67⁺ CTCs

*   *

Ctrl sgRNA   *Il1r1* KO   *Il6st* KO

☐ CTC-neutrophil clusters
☐ CTCs (no WBC)

Fig. 6

a)

b)

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2012142611 A
- US 2016250341 A

- US 2016075767 A
- US 2015368302 A

**Non-patent literature cited in the description**

- **SAMBROOK et al.** Molecular Cloning: A Laboratory Manual. Cold Spring Harbor Laboratory Press, 1989
- **AUSUBEL et al.** Short Protocols in Molecular Biology. John Wiley & Sons, Inc, 1999
- **SKERRA.** *Biochim. Biophys. Acta,* 2000, vol. 1482 (1-2), 337-50
- **KWAN et al.** *Structure,* 2003, vol. 11 (7), 803-813
- Topical and Transdermal Drug Delivery: Principles and Practice. Wiley, 2011, vol. 13
- **GUY ; HANDCRAFT.** Transdermal Drug Delivery Systems: Revised and Expanded. CRC Press, 2002

- Topical Drug Delivery Formulations. CRC Press, 1989
- *CHEMICAL ABSTRACTS,* 914613-48-2
- *CHEMICAL ABSTRACTS,* 375823-41-9
- *CHEMICAL ABSTRACTS,* 541502-14-1
- **LI, H. et al.** *Nat Genet,* 2017, vol. 49, 708-718
- **CRISTOFANILLI, M. et al.** *N Engl J Med,* 2004, vol. 351, 781-791
- **YU et al.** *Science,* 2014, vol. 345, 216-220
- **MACAULAY et al.** *Nat Protoc,* 2016, vol. 11, 2081-2103
- **CHEN et al.** *Cell,* 2015, vol. 160, 1246-1260
- **LI et al.** *Nat Genet,* 2017, vol. 49, 708-718